# EUROPÄISCHE PATENTSCHRIFT

(11) **EP 2 134 831 B1**
(45) Veröffentlichungstag und Bekanntmachung des Hinweises auf die Patenterteilung: **26.04.2017**
(21) Anmeldenummer: 08714237.8
(22) Anmeldetag: 28.01.2008
(51) Int. Cl.: C12M 3/00, A61M 5/00

(54) **VORRICHTUNG UND VERFAHREN ZUR DEPOSITION VON BIOLOGISCHEM MATERIAL IN EINEM ZIELSUBSTRAT**
APPARATUS AND METHOD FOR THE DEPOSITION OF BIOLOGICAL MATERIAL IN A TARGET SUBSTRATE
PROCÉDÉ ET DISPOSITIF DE DÉPÔT DE MATIÈRE BIOLOGIQUE DANS UN SUBSTRAT CIBLE

(30) Priorität: 31.01.2007 DE 102007004855
(43) Veröffentlichungstag der Anmeldung: 23.12.2009
(73) Patentinhaber: Fraunhofer-Gesellschaft zur Förderung der angewandten Forschung e.V., 80686 München (DE)
(72) Erfinder: ZIMMERMANN, Heiko, 66386 St. Ingbert (DE); FUHR, Günter, R., 13187 Berlin (DE)
(74) Vertreter: Hertz, Oliver
(86) Internationale Anmeldenummer: PCT/EP2008/000642
(87) Internationale Veröffentlichungsnummer: WO 2008/092619

(56) Entgegenhaltungen:
- EP-A- 0 331 855
- EP-A- 1 637 173
- EP-A- 1 649 885
- WO-A-94/07603
- WO-A2-98/10750
- DE-T2- 69 732 106
- US-A- 5 036 006
- US-A- 5 219 746
- US-A1- 2005 018 036
- US-A1- 2005 226 855

## Beschreibung

Die Erfindung betrifft eine Vorrichtung, die zur Deposition von biologischem Material in einem Substratmaterial eingerichtet ist, insbesondere eine Depositionsvorrichtung, die zur Einbettung von biologischen Zellen oder Zellbestandteilen in dem Substratmaterial eingerichtet ist. Des Weiteren betrifft die Erfindung Verfahren und Anwendungen der Deposition von biologischem Material in einem Substratmaterial.

Aus der Praxis biologischer oder medizinischer Verfahren ist allgemein bekannt, biologische Zellen aus einer Zellkultur oder einem Spenderorganismus zu einem Substrat zu übertragen. Auf dem Substrat werden die Zellen zum Beispiel einem Test oder einer weiteren Kultivierung unterzogen. Das Substrat, zu dem die Zellen übertragen werden, kann ein Teil eines Laborgerätes, wie zum Beispiel ein Kulturgefäß, oder ein biologisches Material, wie zum Beispiel eine Zellkultur oder ein Gewebe in einem biologischen Organismus umfassen. Während für die Deposition biologischer Zellen auf festen Substratoberflächen zahlreiche Techniken (z. B. Dispensiertechniken, Picking-Spotting-Techniken usw.) verfügbar sind, ist die Technik der gezielten und reproduzierbaren Deposition von Zellen oder Zellbestandteilen in biologischem Material nur ungenügend entwickelt.

Zur Einbettung von Zellen im Gewebe eines Organismus zum Beispiel für medizinische Zwecke wird bisher eine Suspension der Zellen direkt in das betreffende Gewebe eingespritzt oder in die Blutbahn des Organismus injiziert. Das Einspritzen von Zellen in Gewebe, z. B. Herzmuskelgewebe hat den Nachteil, dass beim Einspritzen das dichte Material des Gewebes verdrängt werden muss, wobei hohe mechanische Belastungen der suspendierten Zellen auftreten. Des Weiteren ist von Nachteil, dass beim Einspritzen meist erhebliche Flüssigvolumenanteile in das Gewebe eingebracht werden, die unerwünscht sind oder eine Störung im Gewebe darstellen. Schließlich ergibt das Einspritzen undefinierte Depositionen, da die Verteilung der Zellen im Gewebe kaum beeinflusst werden kann. Die Injektion der Zellen in die Blutbahn ermöglicht zwar einen schonenden Transfer in den Organismus. Das Problem der geometrisch undefinierten Deposition vergrößert sich jedoch noch. In der Blutbahn kann es durch eine Verdriftung der Zellen in Teile des Organismus außerhalb des Zielgewebes zu unerwünschten Nebenwirkungen, bis hin zur Tumorbildung kommen.

Bei der Injektion von Stammzellen oder Vorläuferzellen in eine Zellkultur oder einen biologischen Organismus sind zusätzliche Anforderungen gegeben, die mit dem herkömmlichen Verfahren nur ungenügend erfüllt werden. Stammzellen stellen extrem sensible Zelltypen dar, deren Zustand und Differenzierungspotenzial durch mechanische und biochemische Umgebungsbedingungen und insbesondere Oberflächenkontakte beeinflusst werden. Eine wirkungsvolle Therapie mit Stammzellen, die beispielsweise im Zielgewebe in einen bestimmten Zelltyp differenzieren sollen, erfordert einen schonenden Transfer und eine reproduzierbare Deposition im Zielgewebe. Eine örtlich reproduzierbare Deposition erfordert nicht nur die Ablage der Zellen im gewünschten Zielgewebe, sondern auch die genaue Einstellung der Zahl, Tiefenposition und/oder gegenseitigen Abstände der eingebetteten Zellen im Zielgewebe.

In WO 2004/074426 wird ein Verfahren zur schonenden Übertragung biologischer Zellen in biologisches Gewebe unter Verwendung eines verletzungsfrei verdrängenden Transferwerkzeugs beschrieben. Diese Technik kann in der Praxis nachteilig sein, wenn große Zellzahlen übertragen werden sollen. Beispielsweise kann in der regenerativen Medizin die Aufgabe bestehen, 10⁶ bis 10⁸ Zellen und entsprechend ein Suspensionsvolumen von einigen Millilitern in ein Gewebe einzubringen. Da die Positionierung des Transferwerkzeugs extrem zeitaufwändig ist, kann die in WO 2004/074426 beschriebene Technik für eine routinemäßige Deposition derart großer Zellzahlen ungeeignet sein.

Aus der EP 1637173 A2 ist ein Verfahren bekannt, genetisches Material ohne Nadel durch eine Haut in biologisches Gewebe einzuführen. Vor allem sollen sehr kleine Partikel, welche die Hautzellen nicht beschädigen, in das Innere von Zellen eingeführt werden. Transportiert werden die Partikel durch einen Gasfluss. Auch in der DE 69732106 T2 wird die Zuführung von kleinen Partikeln in das Innere von lebenden Zellen beschrieben, wobei die Partikel zunächst in einer Trägerpatrone haftend angeordnet werden und dann durch einen Luftstrom in das Substrat befördert werden.

Grundsätzlich ist aus der Praxis biologischer Verfahren die Verwendung von gefrorenen biologischen Materialien bekannt. In der WO 94/07603 ist ein Verfahren zur Kryobefestigung von biologischen Materialien an einem Festkörper mit Hilfe eines Druckluftgerätes offenbart. Aus der US 2005/0214946 A1 sind in einer Küvette eingefrorene Zellen für Elektroporationsanwendungen bekannt.

In der US 5219746 A und der US 5036006 sind Verfahren beschrieben, wie kleine Partikel durch Luftdruck in biologisches Gewebe transportiert werden. Dabei werden auch biologische Partikel in gefrorener Form verabreicht. Beide Verfahren zielen auf den Transport von kleinen Partikeln mit Durchmessern im Bereich von 10 nm bis zu wenigen Mikrometern in das Innere von Zellen ab, ohne dabei die Zellen im Zielsubstrat zu zerstören. Ein Transport oder eine Deposition von größeren Zellen oder ganzen Zellgruppen ist damit nicht möglich.

Die oben genannten Probleme treten nicht nur bei der Deposition von Stammzellen, sondern auch bei der Einführung von anderen Zelltypen, Zellbestandteilen oder Wirkstoffen auf, wenn ein verlustfreier, schonender Transfer unter vorgebbaren geometrischen oder quantitativen Bedingungen erforderlich ist. Beispielsweise stellt die gezielte Deposition von Wirkstoffen, wie zum Beispiel Differenzierungs- oder Wachstumsfaktoren im Gewebe ein bisher ungelöstes Problem dar. Des Weiteren bestehen die genannten Probleme nicht nur bei der Deposition biologischen Materials in einem Organismus, sondern auch bei der Einbettung von biologischem Material in biologische oder nicht-biologische Substratmaterialien außerhalb eines Organismus, wie zum Beispiel in eine Zellkultur.

Aus US 2005/0018036 A1 ist eine Laser-Ablationstechnik bekannt, bei der Laserlicht in eine Trägerschicht fokussiert wird, in der absorbierte Energie in Wärme konvertiert wird, so dass ein Teil einer Transferschicht aus biologischem Material, die den bestrahlten Abschnitt der Trägerschicht bedeckt, von der Transferschicht getrennt und in einen angrenzenden Raum übertragen wird. In WO 98/10750 A2 wird ein Makromolekültransfer in ein Gewebe mit einem ohne Injektionsnadel bei Raumtemperatur arbeitenden Gerät beschrieben.

Die Aufgabe der Erfindung ist es, eine verbesserte Depositionsvorrichtung bereitzustellen, die zur Deposition biologischen Materials in ein Zielsubstrat eingerichtet ist und mit der Nachteile herkömmlicher Depositionstechniken überwunden werden. Die Aufgabe der Erfindung ist es des Weiteren, ein verbessertes Verfahren zur Deposition biologischen Materials in einem Zielsubstrat bereitzustellen, mit dem Nachteile der herkömmlichen Techniken überwunden werden.

Diese Aufgaben werden durch eine Depositionsvorrichtung und ein Verfahren zur Deposition biologischen Materials mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt basiert die Erfindung auf der allgemeinen technischen Lehre, eine Depositionsvorrichtung bereitzustellen, die zur Deposition von biologischem Material in einem Zielsubstrat eingerichtet ist und insbesondere eine Antriebseinrichtung aufweist, mit der das biologische Material durch Ausübung einer Antriebskraft in das Zielsubstrat eingeführt werden kann. Die Depositionsvorrichtung ist ferner insbesondere mit einer Ladeeinrichtung ausgestattet, die zur Bereitstellung des biologischen Materials an der Antriebseinrichtung eingerichtet ist. Im Unterschied zu herkömmlichen Techniken zur Übertragung biologischen Materials ist die Antriebseinrichtung so gebildet, dass das biologische Material in einem gefrorenen Zustand beschleunigt wird und zumindest ein Teil des biologischen Materials unter der Wirkung der Antriebskraft auf das biologische Material in das Zielsubstrat eingebettet wird.

Gemäß einem zweiten Gesichtspunkt basiert die Erfindung auf der allgemeinen technischen Lehre, ein Verfahren zur Deposition biologischen Materials in einem Zielsubstrat bereitzustellen, wobei sich das Verfahren insbesondere dadurch auszeichnet, dass das biologische Material mit der erfindungsgemäßen Depositionsvorrichtung unter der Wirkung einer Antriebskraft in einem gefrorenen Zustand in das Zielsubstrat eingeführt wird.

Die Erfinder haben festgestellt, dass die Probleme der herkömmlichen Techniken effektiv gelöst werden können, indem das biologische Material im gefrorenen Zustand mit einer derart hohen Geschwindigkeit zu dem Zielsubstrat bewegt wird, dass ausschließlich das gefrorene biologische Material unmittelbar in das Material des Zielsubstrats eindringt. Der Weg des biologischen Materials wird durch Verdrängung im Zielsubstrat gebildet. Das biologische Material im gefrorenen Zustand ist ein festes Material, das mindestens einen festen Partikel umfasst. Der feste Zustand ermöglicht eine schonende Einbettung des biologischen Materials in das Zielsubstrat. Des Weiteren wird durch den festen Zustand sichergestellt, dass während der Bewegung des biologischen Materials zum Zielsubstrat und während des Eindringens des biologischen Materials in das Zielsubstrat die Masse und Form des biologischen Materials unverändert bleibt. Vorteilhafterweise können somit der Depositionsort und die Depositionsmenge durch die Einstellung der Antriebskraft (insbesondere Richtung und/oder Betrag der Antriebskraft) reproduzierbar eingestellt werden. Das biologische Material kann erfindungsgemäß verkapselungsfrei in das Zielsubstrat eingeführt werden. Vorteilhafterweise ermöglicht die Erfindung somit eine rückstandsfreie Einbettung des biologischen Materials im Zielsubstrat. Ein wesentlicher Vorteil der Erfindung wird erreicht, wenn das biologische Material mindestens eine biologische Zelle enthält. Biologische Zellen sind im gefrorenen Zustand nicht aktiv und nicht gegenüber Umgebungseinflüssen sensitiv. Entsprechend können sie beim Durchgang durch das Gewebe bei der Einbettung keine biochemischen Signale aufnehmen. Vorteilhafterweise wird das biologische Material unverändert eingebettet, d.h. der biochemische Zustand des biologischen Materials vor der erfindungsgemäßen Einbettung ist identisch mit dem biochemischen Zustand des biologischen Materials bei oder unmittelbar nach der Deposition im Zielsubstrat. Dieser Vorteil ist bei der Deposition von Stammzellen besonders wichtig. Stammzellen zeichnen sich dadurch aus, dass bei Oberflächenberührungen Signalkaskaden ausgelöst werden können, die den Zellzustand und schließlich eine Differenzierung der Stammzelle bestimmen. Während bei den herkömmlichen Techniken, bei denen lebende, nichtgefrorene Zellen in Gewebe eingeführt werden, die Zellen vielen biochemischen Signalen ausgesetzt sind, die unerwünscht oder zumindest unkontrollierbar sind, werden beim erfindungsgemäßen Verfahren biochemische Signale während der Einbettung vermieden. Erst nach dem Auftauen im Zielsubstrat können Kontakte der eingebetteten Stammzelle mit der Umgebung, z.B. mit biologischen Zellen in einem Gewebe erfolgen und eine gewünschte Entwicklung der Stammzelle auslösen.

Die mit der Antriebseinrichtung ausgeübte Antriebskraft wird in Abhängigkeit von der konkreten Anwendung der Erfindung so gewählt, dass das biologische Material eine ausreichend hohe Geschwindigkeit erhält, um im Zielsubstrat in die Oberfläche und/oder in die gewünschte Tiefe einzudringen. Die Geschwindigkeit wird gemäß der Erfindung so gewählt, dass das biologische Material eine kinetische Energie erhält, die gleich der Verdrängungsarbeit ist, welche das biologische Material bis zum Erreichen der gewünschten Tiefe im Zielsubstrat verrichtet. Die erforderliche Verdrängungsarbeit ist vom Material des Zielsubstrats, z. B. von der Art des biologischen Gewebes abhängig und kann beispielsweise durch einfache Testreihen ermittelt werden. Vorzugsweise wird das gefrorene biologische Material auf eine Geschwindigkeit beschleunigt, die mindestens 0,5 m/s, besonders bevorzugt mindestens 2 m/s beträgt. Die Geschwindigkeit wird z. B. mit der Geschwindigkeit eines Druckfluids und/oder eines Kühlmediums eingestellt, mit dem das biologische Material in der Antriebseinrichtung beaufschlagt wird.

Weitere Vorteile der Erfindung bestehen darin, dass vorbestimmte Zahlen biologischer Zellen im Zielsubstrat deponierbar sind. Dabei können das deponierte Volumen und/oder die Belastung (Stress durch Scherkräfte) während der Deposition minimiert werden. Die Deposition des biologischen Materials kann mit einer örtlichen Auflösung im Sub-Millimeterbereich in lateraler und/oder transversaler Richtung realisiert werden. Ohne Einschränkung der örtlichen Depositionsgenauigkeit kann die Depositionsvorrichtung (oder zumindest ein Teil von dieser) relativ zum Zielsubstrat im Millimeter- oder sogar im Zentimeter-Bereich Verfahren werden, um das biologische Material in einem relativ großen Zielsubstrat einzubetten. Ein weiterer Vorteil besteht in der hohen Depositionsgeschwindigkeit. Die Einbettung einer Vielzahl biologischer Zellen in ein Zielsubstrat kann innerhalb weniger Sekunden oder Minuten erfolgen, was insbesondere für *in vitro*-Anwendungen der erfindungsgemäßen Deposition von Vorteil ist.

Das eingebettete biologische Material bildet mit dem umgebenden Zielsubstrat einen Materialschluss, d.h. das eingebettete biologische Material wird im eingebetteten Zustand allseitig vom Material des Zielsubstrats berührt. Vorteilhafterweise ermöglicht dies ein schnelles Auftauen nach der Deposition und einen schnellen Abtransport von einem ggf. im biologischen Material enthaltenen Kryoprotektivum bereits beim Auftauen.

Mit dem Begriff "biologisches Material" wird hier jedes Material bezeichnet, das mindestens eine biologische Zelle, mindestens eine Zellgruppe, mindestens ein Zellbestandteil, mindestens ein biologisches Makromolekül, wie zum Beispiel Enzyme, Proteine, Aminosäuren, DNS oder RNS, mindestens einen Wirkstoff (biologisch oder medizinisch wirksame Zusatzsubstanz) und/oder mindestens einen biokompatiblen Zusatzstoff, wie zum Beispiel einen Füllstoff, eine Suspensionsflüssigkeit, oder ein Kultivierungsmedium, enthält. Das biologische Material hat bei Raumtemperatur oder einer Kultivierungstemperatur (oberhalb 0°C) einen flüssigen, fließfähigen oder deformierbaren Zustand. Besonders bevorzugt sind Ausführungsformen der Erfindung, bei denen das biologische Material mindestens eine biologische Zelle enthält, da sich bei dieser die Vorteile der schonenden Einbettung im gefrorenen Zustand besonders ausprägen. Bei der mindestens einen biologischen Zelle kann es sich beispielsweise um eine eukaryotische Zelle handeln. Im biologischen Material ist insbesondere mindestens einer der Zelltypen enthalten, die Stammzellen, Vorläuferzellen, differenzierte Zellen, Tumor-beeinflussende Zellen, Nervenzellen, Muskelzellen, insbesondere Herzmuskelzellen, Knorpelzellen, Knochenzellen, Inselzellen, Drüsenzellen, Endothelzellen und Epithelzellen umfassen. Die mindestens eine biologische Zelle bildet vorteilhafterweise mit der Zusatzsubstanz eine Zusammensetzung, in der die Zelle oder Zellgruppe von der Zusatzsubstanz oder umgekehrt die Zusatzsubstanz von einer Zellgruppe umhüllt ist.

Im gefrorenen Zustand bildet das biologische Material mindestens einen festen Partikel mit einer typischen Querschnittsdimension, die vorzugsweise kleiner als 1 mm, besonders bevorzugt kleiner als 500 µm, insbesondere kleiner 100 µm, wie zum Beispiel 20 µm oder 10 µm oder kleiner ist. Typischerweise ist die Masse der Partikel im Bereich von 0,1 ng bis 10 µg gewählt. Die geringe Partikelgröße ergibt den zusätzlichen Vorteil einer minimalen Verwundung des Zielsubstrats bei der Einführung des biologischen Materials. Im Zielsubstrat werden minimale, bei Verwendung eines deformierbaren Substratmaterials reversibel sich verschließende Einschusskanäle gebildet, die sich nach der Einbettung ohne Hinterlassung einer störenden Wunde verschließen. Der im biologischen Material optional enthaltene biokompatible Zusatzstoff kann eine bei Raumtemperatur feste, flüssige oder gasförmige Substanz enthalten. Das biologische Material kann beispielsweise in ein inertes Gas, z. B. CO₂, eingebettet sein, das im gefrorenen Zustand des biologischen Materials fest ist und nach der Einbettung in das Zielsubstrat rückstandslos in die Gasphase übergeht.

Optional kann das biologische Material zusätzlich ein bei Raumtemperatur festes Trägermaterial enthalten. Das Trägermaterial umfasst z. B. ein Substratmaterial, mit dem das biologische Material durch ein vorhergehendes Kryokonservierungsverfahren verbunden ist.

Mit dem Begriff "Zielsubstrat" wird hier jede Materialzusammensetzung natürlichen oder synthetischen Ursprungs bezeichnet, die zur Einbettung des biologischen Materials eingerichtet ist und das biologische Zellen enthält. Das Zielsubstrat umfasst biologische Zellen z. B. als Teil eines menschlichen oder tierischen Organismus, als Teil einer Pflanze, als Zellgruppe außerhalb des Organismus oder der Pflanze oder als Zellkultur. Alternativ oder zusätzlich kann das Zielsubstrat ein festes, deformierbares Substratmaterial, insbesondere ein Kultivierungsgel, wie zum Beispiel Agar-Gel, oder eine zähflüssige Kultivierungsflüssigkeit umfassen. Im Zielsubstrat sind Zellen enthalten und die Größe der gefrorenen Partikel aus biologischem Material wird vorzugsweise nahezu gleich der Größe der Zellen oder größer gewählt.

Mit dem Begriff "Deposition" des biologischen Materials in dem Zielsubstrat wird die Einbettung oder Einführung des biologischen Materials in das Material des Zielsubstrats bezeichnet. Die Deposition umfasst eine vollständige Einbettung, bei der das biologische Material allseitig vom Material des Zielsubstrats umgeben ist, oder eine teilweise Einbettung, bei der das biologische Material hin zu zumindest einer Seite in die Oberfläche des Zielsubstrats eingebettet ist. Die Einführung des biologischen Materials wird auch als Schießen des mindestens einen festen Partikels des biologischen Materials in das Zielsubstrat bezeichnet. Das gefrorene Partikelmaterial wird bei der Verabreichung mit Hilfe z. B. eines komprimierten Gases auf hohe Geschwindigkeit gebracht und dringt auf Grund seiner Massenträgheit in das Zielsubstrat ein.

Mit dem Begriff "Ladeeinrichtung" wird eine Komponente bezeichnet, die zur Bereitstellung des biologischen Materials in einem Zustand eingerichtet ist, in dem die Beaufschlagung mit der Antriebskraft vorgesehen ist.

Vorteilhafterweise kann die erfindungsgemäße Deposition biologischen Materials mit verschiedenen Typen von Antriebskräften realisiert werden. Gemäß der Erfindung ist ein Fluidantrieb vorgesehen, der zur Erzeugung der Antriebskraft durch Ausübung eines Gasdruckes auf das biologische Material eingerichtet ist. Vorteilhafterweise sind Druckwellen verwendbar, die für andere technische Anwendungen, wie zum Beispiel die Materialbearbeitung mit beschleunigten CO₂-Kristallen oder das spritzenfreie Impfen bekannt sind. Ein weiterer Vorteil des Fluid-antriebs besteht in der hohen Effektivität der Kraftübertragung, die insbesondere hohe Eindringtiefen im Zielsubstrat bis zu 5 cm ermöglicht. Der Fluidantrieb umfasst zum Beispiel eine Druckquelle mit einem steuerbaren Ausgangsventil.

Der Fluidantrieb enthält vorzugsweise eine Beschleunigungsleitung, in der das Fluid von der Druckquelle strömt. In der Beschleunigungsleitung wird das biologische Material der Antriebskraft ausgesetzt. Vorteilhafterweise wird durch die longitudinale Ausdehnung der Beschleunigungsleitung dem biologischen Material eine vorbestimmte Bewegungsrichtung aufgeprägt, so dass durch die Ausrichtung der Beschleunigungsleitung relativ zum Zielsubstrat der Depositionsort des biologischen Materials bestimmt werden kann.

Es wird auch ein Translationsantrieb beschrieben (keine Ausführungsformen der Erfindung), der ein mechanisches Antriebselement aufweist. Das mechanische Antriebselement ist zur unmittelbaren Ausübung der Antriebskraft auf das biologische Material eingerichtet. Das mechanische Antriebselement umfasst einen Träger, z. B. in Form einer Platte, eines Stabes oder eines Gitters, zur Aufnahme des biologischen Materials, wobei der Träger mit einer Schwingungsquelle, z. B. einer elektromagnetischen oder piezoelektrischen Schwingungsquelle zu Schwingungen anregbar ist. Durch die Schwingung wird auf das biologische Material eine Abstoßungskraft ausgeübt, unter deren Wirkung das biologische Material zum Zielsubstrat beschleunigt wird.

Der Translationsantrieb enthält eine Trägerplattform, die zur Aufnahme von hängenden Tropfen des biologischen Materials, insbesondere hängenden Tropfen eines Kultivierungsmediums, in dem biologische Zellen suspendiert sind, eingerichtet ist. Hängende Tropfen bilden schonende Kultivierungsbedingungen für empfindliche Zellen, wie zum Beispiel Vorläuferzellen oder Stammzellen. Die Trägerplattform ermöglicht die Erhaltung der Kultivierungsbedingungen bis zu einer Abkühlung und Überführung in den gefrorenen Zustand zu Beginn der Deposition.

Gemäß einer bevorzugten Variante der Erfindung wird das biologische Material über einen freien Abstand von der Depositionsvorrichtung zu dem Zielsubstrat bewegt. Unter der Wirkung der Antriebskraft fliegt das biologische Material über eine freie Wegstrecke, deren Länge im Bereich von Bruchteilen eines mm bis zu 50 cm gewählt sein kann. Besonders bevorzugt ist eine freie Wegstrecke, deren Länge im Bereich von 1 mm bis 10 cm gewählt ist und zum Beispiel 0,5 cm bis 2 cm beträgt. Diese Wegstrecken haben sich für eine störungsfreie Einbettung als besonders vorteilhaft erwiesen. Vorteilhafterweise kann mit der Richtung der Wegstrecke auch der Depositionsort im Zielsubstrat beeinflusst werden. Alternativ kann die Depositionsvorrichtung zur Übertragung des biologischen Materials auf das Zielsubstrat aufgesetzt werden.

Gemäß einer bevorzugten Ausführungsform der Erfindung ist die Depositionsvorrichtung mit einer Präparationseinrichtung ausgestattet, die zur Vorbereitung des biologischen Materials und zu dessen Bereitstellung an der Ladeeinrichtung vorgesehen ist. Vorteilhafterweise können physikalische und/oder chemische Eigenschaften des biologischen Materials unmittelbar vor dessen Übertragung im gefrorenen Zustand zu dem Zielsubstrat eingestellt werden. Physikalische Eigenschaften umfassen zum Beispiel die Größe (Volumen, Masse) von Suspensionstropfen und/oder die Zahl der in einem Suspensionstropfen enthaltenen Zellen. Chemische Eigenschaften umfassen zum Beispiel die Anteile eines Kryoprotektivums im Suspensionstropfen.

Vorteilhafterweise sind verschiedene Varianten der Erfindung möglich, die sich in Bezug auf den Zeitpunkt der Überführung des biologischen Materials in den gefrorenen Zustand unterscheiden. Erstens kann das biologische Material in flüssiger Form bereitgestellt und erst an der Ladeeinrichtung und/oder während der Bewegung zum Zielsubstrat in den gefrorenen Zustand überführt werden. In diesem Fall umfasst die Präparationseinrichtung vorzugsweise einen Tropfengenerator, der zur Bildung voneinander abgegrenzter Tropfen des biologischen Materials eingerichtet ist. Zweitens kann erfindungsgemäß vorgesehen sein, dass das biologische Material bereits in der Präparationseinrichtung in den gefrorenen Zustand überführt wird. In diesem Fall umfasst die Präparationseinrichtung vorzugsweise einen Partikelgenerator, der zur Erzeugung von gefrorenen Tropfen des biologischen Materials eingerichtet ist. Diese Variante der Erfindung hat den besonderen Vorteil, dass das biologische Material sich nach der Präparation und Bereitstellung an der Ladeeinrichtung nicht mehr verändern kann. Partikel können beispielsweise erzeugt werden, indem Tropfen aus einer Zellsuspension, die sich auf einer hydrophoben Unterlage bilden, eingefroren werden.

Allgemein kann durch die Einstellung der Antriebskraft mit der Antriebseinrichtung der Depositionsort des gefrorenen biologischen Materials bestimmt werden. Wenn die erfindungsgemäße Depositionsvorrichtung jedoch mit einer Lenkeinrichtung ausgestattet ist, die zur Einstellung der Bewegungsrichtung des biologischen Materials eingerichtet ist, können sich Vorteile durch eine verbesserte Depositionsgenauigkeit ergeben. Die Lenkeinrichtung weist vorzugsweise eine Düseneinrichtung mit einer oder mehreren Ausgangsdüsen auf. Jede Ausgangsdüse bildet das Ende eines Düsenkanals, dessen longitudinale Richtung die Bewegungsrichtung des biologischen Materials bestimmt. Vorteilhafterweise kann die Düseneinrichtung zur Bewegung des biologischen Materials zu dem Zielsubstrat auf Bahnen eingerichtet sein, die ein vorbestimmtes geometrisches Muster bilden. Erfindungsgemäß können gefrorene Partikel des biologischen Materials zeitgleich an verschiedenen Depositionsorten im Zielsubstrat eingebettet werden. Vorteilhafterweise wird damit eine hohe Parallelität der Deposition zum Beispiel von biologischen Zellen und damit eine hohe Depositionsgeschwindigkeit erreicht.

Wenn die Lenkeinrichtung Teil eines manuell handhabbaren Werkzeugs ist, können sich Vorteile für eine flexible Ausrichtung der Bewegungsbahn des biologischen Materials relativ zum Zielsubstrat durch eine Person ergeben, die die Depositionsvorrichtung bedient. Vorzugsweise ist die Lenkeinrichtung relativ zu den übrigen Komponenten der Depositionsvorrichtung beweglich. Beispielsweise kann ein behandelnder Arzt die Lenkeinrichtung manuell wie ein Operationswerkzeug oder eine Bestrahlungsquelle in geeigneter Weise relativ zum Zielsubstrat orientieren.

Gemäß einer bevorzugten Ausführungsform der Erfindung umfasst die Ladeeinrichtung einen Probeninjektor, der zur Bereitstellung des biologischen Materials in der Antriebseinrichtung, insbesondere in der Beschleunigungsleitung der Antriebseinrichtung eingerichtet ist. Mit dem Probeninjektor kann die Menge des biologischen Materials festgelegt werden, das an einen bestimmten Depositionsort im Zielsubstrat eingebettet werden soll. Vorteilhafterweise können verschiedene Typen von Probeninjektoren verwendet werden, die an die jeweilige Form des biologischen Materials bei Bereitstellung in der Antriebseinrichtung angepasst sind.

Gemäß einer ersten Variante der Erfindung kann der Probeninjektor eine Injektorleitung aufweisen, die in die Beschleunigungsleitung der Antriebseinrichtung mündet. Durch die Injektorleitung kann die biologische Probe in die Beschleunigungsleitung eingeführt werden. Der Transport der biologischen Probe in der Injektorleitung erfolgt beispielsweise durch einen Druck, der an der Ladeeinrichtung und/oder Präparationseinrichtung ausgeübt wird. Vorzugsweise weist die Injektorleitung ein steuerbares Injektorventil auf, mit dem die Zuführung des biologischen Materials in die Beschleunigungsleitung einstellbar ist.

Gemäß einer weiteren Variante weist der Probeninjektor einen Tropfendispenser auf, der zur Zuführung von flüssigen Tropfen des biologischen Materials in die Beschleunigungsleitung oder auf die Trägerplattform eingerichtet ist. Der Tropfendispenser kann beispielsweise aufgebaut sein, wie es aus der herkömmlichen Dispensiertechnik für Screening-Methoden in der Biologie oder Medizin bekannt ist. Gemäß einer alternativen Variante kann der Probeninjektor einen Partikeldispenser aufweisen, mit dem feste Partikel des gefrorenen biologischen Materials in die Beschleunigungsleitung injizierbar sind.

Besondere Vorteile für eine breite und flexible Anwendung der erfindungsgemäßen Deposition ergeben sich, wenn der Probeninjektor eine Injektorpatrone aufweist, die in die Beschleunigungsleitung der Antriebseinrichtung einsetzbar ist. Die Injektorpatrone umfasst eine Hülse, die zur Aufnahme von Partikeln gefrorenen Materials eingerichtet ist, mit Abdeckelementen an Ein- und Ausströmöffnungen. Die Injektorpatrone weist in Längsrichtung die Einströmöffnung und die Ausströmöffnung auf, die zur Durchströmung mit Fluid des Fluidantriebs eingerichtet sind. Bei Durchströmung des Fluids durch die Injektorpatrone wird das biologische Material der Antriebskraft ausgesetzt und mit dem Fluid zum Zielsubstrat bewegt. Vorzugsweise ist die Injektorpatrone in der Beschleunigungsleitung austauschbar angeordnet. Vorteilhafterweise kann eine beladene Injektorpatrone in die Beschleunigungsleitung eingesetzt und zur Deposition des biologischen Materials mit dem Fluid durchströmt werden. Nach Beendigung der Deposition kann die Injektorpatrone aus der Beschleunigungsleitung entfernt und ggf. durch eine weitere Injektorpatrone ersetzt werden.

Vorzugsweise ist mindestens eine der Einström- und Ausströmöffnungen mit einem Abdeckelement versehen, durch das die Injektorpatrone geschlossen ist und dass bei Ausübung der Antriebskraft, z. B. durch das strömende Fluid geöffnet, z. B. durchbrochen werden kann. Vorteilhafterweise wird damit ein verbesserter Schutz des biologischen Materials beim Transport der Injektorpatrone vor dem Einsetzen in die Depositionsvorrichtung erreicht.

Die Lade- und Präparationseinrichtungen, die oben getrennt beschrieben werden, können alternativ durch eine gemeinsame Komponente der erfindungsgemäßen Depositionsvorrichtung gebildet werden. In diesem Fall wird z. B. der Tropfengenerator zugleich als Tropfendispenser zur Bereitstellung des biologischen Materials an der Antriebseinrichtung verwendet.

Gemäß der Erfindung ist vorgesehen, dass die Depositionsvorrichtung mit mindestens einer Kühleinrichtung ausgestattet ist, die zur Überführung des biologischen Materials in den gefrorenen Zustand eingerichtet ist. In Abhängigkeit von den verschiedenen Bauformen der Depositionsvorrichtung kann die Kühleinrichtung als eigenständige Komponente oder als Teil der Lade-, Präparations- und/oder Antriebseinrichtung vorgesehen sein. Ein wesentlicher Vorteil der Kühleinrichtung ist, dass das biologische Material zeitlich unmittelbar vor und/oder während der Beschleunigung zum Zielsubstrat im gekühlten Zustand bereitgestellt und/oder in diesen überführt werden kann.

Wenn gemäß einer ersten Variante die Kühleinrichtung zur Kühlung mindestens eines Teils der Ladeeinrichtung eingerichtet ist, ergeben sich Vorteile durch die Überführung des biologischen Materials in den gefrorenen Zustand unmittelbar vor oder während der Bereitstellung an der Antriebseinrichtung. Erfindungsgemäß ist die Kühleinrichtung zur Kühlung mindestens eines Teils der Antriebseinrichtung eingerichtet, woraus sich weitere Vorteile für eine besonders kurzzeitige Abkühlung des biologischen Materials ergeben. Die Kühleinrichtung ist erfindungsgemäß zur Bereitstellung eines Gases zur Übertragung der Antriebskraft bei einer Temperatur unterhalb des Gefrierpunkts des biologischen Materials eingerichtet. Somit erfüllt die Antriebseinrichtung vorteilhafterweise die Funktionen der Abkühlung und des Antriebs der biologischen Probe.

Gemäß einer weiteren Modifikation der Erfindung kann die Kühleinrichtung zur Kühlung mindestens eines Teils der Lenkeinrichtung, z. B. zur Kühlung der Düseneinrichtung vorgesehen sein. Vorteilhafterweise wird damit die Funktionssicherheit der Depositionsvorrichtung erhöht.

Eine weitere vorteilhafte Ausführungsform der erfindungsgemäßen Depositionsvorrichtung zeichnet sich durch einen Regelkreis aus, der mit der Antriebseinrichtung verbunden ist. Vorteilhafterweise kann mit dem Regelkreis in Abhängigkeit von Depositionsparametern, wie zum Beispiel einem gemessenen Depositionsort oder gemessenen Partikelgrößen die Antriebskraft eingestellt werden. Die Einstellung der Antriebskraft ergibt eine erhöhte Genauigkeit und Reproduzierbarkeit der Einbettung des biologischen Materials im Zielsubstrat.

Gemäß einer weiteren vorteilhaften Ausführungsform der Erfindung ist die Depositionsvorrichtung für einen Schaltbetrieb eingerichtet. Die Lade-, Antriebs-, Präparations- und/oder Kühleinrichtung sind gezielt ein- und ausschaltbar. Vorteilhafterweise können damit vorbestimmte Depositionsprotokolle realisiert werden, mit denen insbesondere die Art des biologischen Materials und der zugehörige Depositionsort festgelegt wird. Besonders bevorzugt sind die Lade- und Antriebseinrichtung für einen synchronisierten Schaltbetrieb eingerichtet. Beide Komponenten, insbesondere die Injektor- und Fluidventile sind gemeinsam schaltbar, um eine Deposition des biologischen Materials zu realisieren.

Weitere Vorteile für die Funktionalität der Depositionsvorrichtung ergeben sich, wenn diese mit mindestens einer der folgenden Zusatzkomponenten ausgestattet ist. Mit einer Abbildungseinrichtung, die vorzugsweise mindestens eine von einer Kameraeinrichtung, einer Mikroskopieeinrichtung, einer Ultraschalleinrichtung, einer Stroboskopeinrichtung und einer Endoskopieeinrichtung umfasst, können vorteilhafterweise Bilder der Depositionsvorrichtung und/oder des Zielsubstrats aufgenommen werden. Die aufgenommenen Bilder, die zum Beispiel Bilder der Oberfläche oder des Volumens des Zielsubstrats oder Bilder des aus der die Positionsvorrichtung austretenden biologischen Materials umfassen, ermöglichen eine Steuerung der Einbettung des biologischen Materials in das Zielsubstrat und/oder eine Bewertung des Depositionsergebnis. Des Weiteren ermöglicht eine Navigationseinrichtung, die zur Bewegung und Fixierung der Depositionsvorrichtung in Bezug auf das Zielsubstrat eingerichtet ist, eine erhöhte Depositionsgenauigkeit. Schließlich kann eine Konditioniereinrichtung, die zum Beispiel zur Einstellung vorbestimmter Beleuchtungs- und/oder Temperaturbedingungen am Zielsubstrat eingerichtet ist, zur Beobachtung des Depositionsergebnisses oder zur Beeinflussung des Auftauens des biologischen Materials im Zielsubstrat verwendet werden. Als Konditioniereinrichtung kann zum Beispiel ein Auftausystem vorgesehen sein, mit dem die Überführung der biologischen Probe in den nichtgefrorenen Zustand im Zielsubstrat beschleunigt wird.

Es wird auch die Verwendung von biologischem Material im gefrorenen Zustand zur Modifizierung biologischen Gewebes in einem erfindungsgemäßen Verfahren beschrieben, wobei das biologische Material mindestens eine Zelle, Zellgruppe, Zellbestandteile, biologische Makromoleküle und/oder einen biologisch wirksamen Wirkstoff enthält.

Weitere Einzelheiten und Vorteile der Erfindung werden im Folgenden unter Bezug auf die beigefügten Zeichnungen beschrieben. Es zeigen:
- Figur 1:: eine schematische Illustration einer ersten Ausführungsform der erfindungsgemäßen Depositionsvorrichtung;
- Figur 2:: eine weitere der Depositionsvorrichtung (keine Ausführungsform der Erfindung);
- Figuren 3: und 4: Illustrationen von Einzelheiten von weiteren Ausführungsformen der erfindungsgemäßen Depositionsvorrichtung;
- Figur 5:: eine weitere Depositionsvorrichtung (keine Ausführungsform der Erfindung);
- Figuren 6 und 7:: Einzelheiten der Verwendung einer ersten Ausführungsvariante einer Injektorpatrone;
- Figur 8:: eine schematische Darstellung zur Verwendung einer zweiten Variante der Injektorpatrone;
- Figur 9:: Illustrationen von verschiedenen Varianten von biologischem Material, das erfindungsgemäß zur Einbettung in ein Zielsubstrat verwendet wird;
- Figur 10:: eine schematische Querschnittsansicht eines Zielsubstrats mit eingebettetem biologischem Material;
- Figur 11:: Querschnittsillustrationen von verschiedenen Phasen der Einbettung biologischen Materials im Zielsubstrat; und
- Figuren 12 bis 15:: Illustrationen weiterer Merkmale von bevorzugten Ausführungsformen des erfindungsgemäßen Depositionsverfahrens.

Die Erfindung wird im Folgenden zunächst unter beispielhaftem Bezug auf die Einbettung biologischer Zellen in ein biologisches Gewebe innerhalb oder außerhalb eines biologischen Organismus beschrieben. Die Erfindung ist jedoch nicht auf diese Anwendung beschränkt. Die Einbettung biologischer Materialien in das Volumen oder die Oberfläche anderer Zielsubstrate, wie zum Beispiel Zellkulturen kann in entsprechender Weise realisiert werden. Einzelheiten der Überführung biologischer Materialien in den gefrorenen Zustand, wie zum Beispiel Merkmale des verwendeten Kühlmediums, der Zusatz eines Kryoprotektivums oder die Schritte eines Einfrierverfahrens werden im Folgenden nicht erläutert, da sie als solche aus der herkömmlichen Kryokonservierung biologischer Proben bekannt sind.

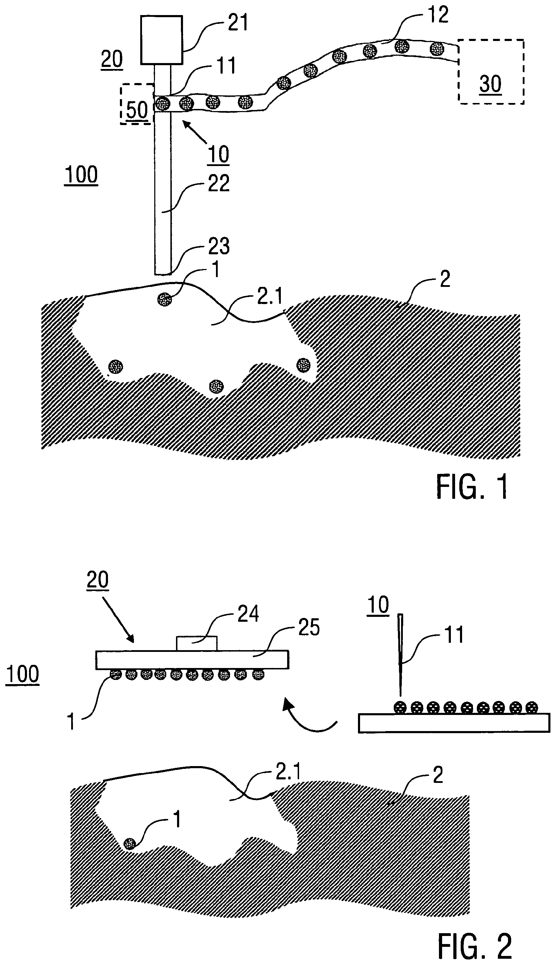

Figur 1 zeigt eine erste Ausführungsform einer erfindungsgemäßen Depositionsvorrichtung 100 in schematischer Schnittansicht. Die Depositionseinrichtung 100 umfasst eine Ladeeinrichtung 10 mit einem Probeninjektor 11 und einer Injektorleitung 12 und eine Antriebseinrichtung 20 mit einer Druckquelle 21 und einer Beschleunigungsleitung 22. Als optionale Komponenten sind zusätzlich eine Präparationseinrichtung 30 und eine Kühleinrichtung 50 gezeigt.

Der Probeninjektor 11 wird im einfachsten Fall durch die Mündung der Injektorleitung 12 in die Beschleunigungsleitung 22 gebildet. Tropfen oder gefrorene Partikel des biologischen Materials 1 werden durch die Injektorleitung 12 unter der Wirkung eines Druckes von der Präparationseinrichtung 30 zum Probeninjektor 11 transportiert. Die Injektorleitung 12 ist ein Rohr oder Schlauch, z. B. aus Glas oder Kunststoff, mit einem Innendurchmesser von zum Beispiel 0,2 mm. In der Injektorleitung 12 werden z. B. flüssige Suspensionstropfen transportiert, zwischen denen Abstände mit Luft oder Inertgas gebildet sind. Alternativ können in der Beschleunigungsleitung 12 gefrorene Tropfen transportiert werden. In diesem Fall besteht die Beschleunigungsleitung vorzugsweise aus einem reibungsarmen Kunststoffmaterial, wie zum Beispiel Teflon.

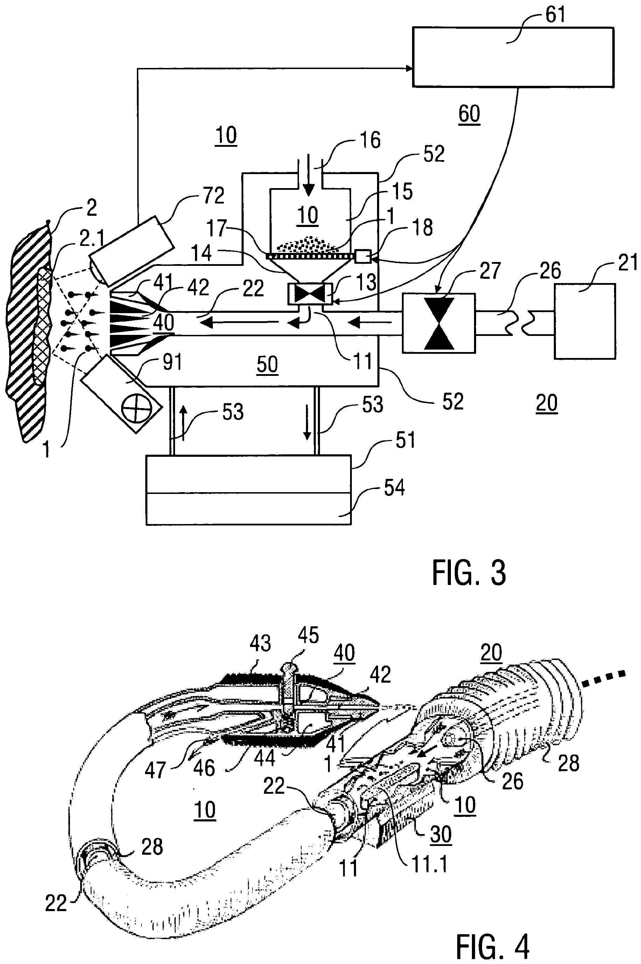

Die Druckquelle 21 ist eine Druckgas-Quelle, die ein Inertgas, wie zum Beispiel Stickstoff enthält und mit einem steuerbaren Fluiddruckventil (siehe Figur 3) ausgestattet ist. Die Beschleunigungsleitung 22 ist eine Hohlleitung, zum Beispiel in Form eines Rohres oder Schlauches, die an einem Ende mit der Druckquelle 21 verbunden ist und ein zweites, freies Ende mit einer Austrittsöffnung 23 aufweist. Die Beschleunigungsleitung 22 ist vorzugsweise zumindest an dem zur Austrittsöffnung 23 weisenden Ende, besonders bevorzugt jedoch vollständig gerade gebildet. Sie besteht zum Beispiel aus Glas oder formstabilen Kunststoff mit einem Innendurchmesser von zum Beispiel 100 µm und einer Länge von zum Beispiel 3 cm. Für eine freie Ausrichtung der Beschleunigungsleitung 22 im Raum kann eine flexible Schlauchverbindung mit der Druckquelle 21 vorgesehen sein, die ortsfest angeordnet ist. Des Weiteren kann für eine manuelle Handhabung der Depositionsvorrichtung 100 auf der Außenseite der Beschleunigungsleitung 22 ein Griffstück (nicht dargestellt) vorgesehen sein.

Die Präparationseinrichtung 30 umfasst einen Tropfen- oder Partikelgenerator, mit dem die Tropfen oder Partikel in der Injektorleitung 12 erzeugt werden. Der Tropfengenerator besteht zum Beispiel aus einer Pumpe, die aus einem Vorratsgefäß mit einer Zellsuspension vorbestimmte Tropfenvolumina in die Injektorleitung 12 fördert.

Die Kühleinrichtung 50 ist beispielhaft am Ort des Probeninjektors 11 gezeigt. Alternativ oder zusätzlich kann die Kühleinrichtung 50 in die Präparationseinrichtung 30 oder in die Antriebseinrichtung 20, speziell in die Druckquelle 21 integriert sein.

Das in Figur 1 beispielhaft illustrierte Zielsubstrat 2 umfasst einen Wundbereich 2.1 innerhalb eines Muskelgewebes. Das Ziel der erfindungsgemäßen Deposition des biologischen Materials 1 besteht in der Einbettung von Epithelzellen oder Vorläuferzellen von Epithelzellen in den Wundbereich 2.1, um die Wundheilung zu fördern.

Zu Deposition des biologischen Materials 1 im Wundbereich 2.1 wird das mit der Ladeeinrichtung 10 in die Antriebseinrichtung 20 eingeführte biologische Material 1 der Antriebskraft ausgesetzt, die durch das Gas, das von der Druckquelle 21 durch die Beschleunigungsleitung 22 strömt, auf das biologische Material 1 ausgeübt wird. Unter der Wirkung der Antriebskraft bewegt sich das biologische Material 1 im gefrorenen Zustand durch die Beschleunigungsleitung 22 zunächst zwischen der Austrittsöffnung 23 und dem Wundbereich 2.1 auf einer freien Flugbahn. Bei Auftreffen auf der Oberfläche des Wundbereichs 2.1 kann das biologische Material 1 aufgrund seiner kinetischen Energie die Zellen des Wundbereichs 2.1 verdrängen und in der gewünschten Depositionstiefe eingebettet werden.

Die in Figur 1 gezeigte Ausführungsform der Depositionsvorrichtung 100 kann dahingehend modifiziert sein, dass die Ladeeinrichtung eine Kombination aus einer Injektorleitung 12 und einem Einführtrichter (siehe Figur 3) umfasst. Gefrorene Partikel des biologischen Materials werden in den Einführtrichter gefüllt und von diesem unter der Wirkung der Gravitation zum Probeninjektor 11 bewegt, um in der Beschleunigungsleitung 22 der Antriebskraft durch das strömende Gas aus der Druckquelle 21 ausgesetzt zu werden.

Bei der in Figur 2 gezeigten Depositionsvorrichtung 100 (keine Ausführungsform der Erfindung) bildet die Antriebseinrichtung 20 einen mechanischen Translationsantrieb mit einem Antriebselement 24 und einer schwingfähigen Trägerplattform 25. Als Ladeeinrichtung 10 ist eine von der Antriebseinrichtung 20 getrennte Komponente vorgesehen, die zur Ablage von Tropfen des biologischen Materials 1 auf der Trägerplattform eingerichtet ist. In diesem Fall wird als Probeninjektor 11 eine Pipette oder ein anderer Tropfendispenser verwendet. Die Bereitstellung des biologischen Materials 1 an der Antriebseinrichtung 20 erfolgt, wie es von der herkömmlichen Kultivierung anhängenden Tropfen an sich bekannt ist. Zunächst werden die Tropfen des biologischen Materials 1 auf der Trägerplattform 25 abgelegt. Anschließend wird die Trägerplattform 25 mit einer schnellen Schwenkbewegung umgekippt, so dass die Suspensionstropfen in den hängenden Zustand überführt werden. In dieser Situation kann zunächst noch eine Kultivierung von Zellen in den hängenden Tropfen vorgesehen sein, beispielsweise um vor der Einbettung des biologischen Materials 1 im Zielsubstrat 2 in jedem Tropfen Zellgruppen zu bilden. Als Zielsubstrat 2 ist beispielsweise analog zu Figur 1 Gewebe mit einem Wundbereich 2.1 vorgesehen, in dem biologischen Material 1 mit Epithel-bildenden Zellen eingebettet werden soll.

Die Deposition des biologischen Materials 1 im Zielsubstrat 2 umfasst die Beaufschlagung des biologischen Materials 1 mit der mechanischen Antriebskraft, die mit dem Translationselement 24 erzeugt und mit der Trägerplattform 25 z. B. durch Schwingungen auf das biologische Material 1 übertragen wird. Anschließend erfolgt die Bewegung des biologischen Materials in einem gefrorenen Zustand in das Zielsubstrat 2. Die Überführung des biologischen Materials 1 in den gefrorenen Zustand kann in Abhängigkeit von der konkreten Anwendung der Erfindung bereits erfolgen, während sich das biologische Material 1 noch an der Trägerplattform 25 befindet. Alternativ oder zusätzlich kann ein Einfrieren auf dem Weg von der Trägerplattform 25 zu Zielsubstrat 2 vorgesehen sein. In beiden Fällen erfolgt das Einfrieren durch Aufblasen von Dampf flüssigen Stickstoffs, der eine Temperatur von -130 °C ausweist.

Bei der in Figur 3 gezeigten Ausführungsform der erfindungsgemäßen Depositionsvorrichtung 100 umfasst die Ladeeinrichtung 10 als Probeninjektor 11 eine Fallöffnung, durch die gefrorene Partikel des biologischen Materials 1 bei Betätigung des Injektorventils 13 aus einem Einführtrichter 14 in die Beschleunigungsleitung 22 der Antriebseinrichtung 20 fallen. Des Weiteren enthält die Ladeeinrichtung einen Vorratsbehälter 15, der über eine Öffnung 16 das bereits gefrorene biologische Material 1 aufnimmt. Das biologische Material 1 kann über die Öffnung 16 einmalig, periodisch oder kontinuierlich von einer Präparationseinrichtung (nicht dargestellt) zugeführt werden, mit der die gefrorenen Partikel des biologischen Materials 1 erzeugt werden. Die Überführung des biologischen Materials 1 vom Vorratsbehälter 15 in den Einführtrichter 14 erfolgt über ein Sieb 17. Durch eine Bewegung des Siebes 17 mit einem Schwingelement 18, z. B. durch Rütteln oder Schwingungen, kann der Durchsatz vom Vorratsbehälter 15 zum Einführtrichter 14 beeinflusst werden.

Die Druckquelle 21 der Antriebseinrichtung 20 ist über eine Druckleitung 26 in Form eines flexiblen Schlauchs und ein Fluiddruckventil 27 mit der Beschleunigungsleitung 22 verbunden. Am Ende der Beschleunigungsleitung 22 ist die Lenkeinrichtung 40 mit einer Düsenanordnung 41 vorgesehen, an der sich der Kanal der Beschleunigungsleitung 22 in eine Vielzahl von einzelnen Düsenkanälen 42 aufspaltet. Die Düsenöffnungen der Düsenkanäle 42 bilden ein vorbestimmtes geometrisches Muster. Die Düsenanordnung 41 ist vorzugsweise an der Beschleunigungsleitung 22 lösbar befestigt und austauschbar. Dies ermöglicht, in Abhängigkeit von den Anforderungen einer konkreten Anwendung eine geeignete Düsenanordnung 41 mit einer passenden Zahl von Düsenkanälen 42 und einen passenden Muster der Düsenöffnungen zu verwenden.

Bei der in Figur 3 gezeigten Ausführungsform der erfindungsgemäßen Depositionsvorrichtung ist die Kühleinrichtung 50 zur Kühlung der Ladeeinrichtung 10, eines Teils der Antriebseinrichtung 20 und der Lenkeinrichtung 40 eingerichtet. Die Kühleinrichtung 50 umfasst ein Kühlmittereservoir 51 und einen Kühlbehälter 52, die über Schläuche 53 miteinander verbunden sind. Das Kühlmittelreservoir 51 ist zur Aufnahme eines flüssigen Kühlmittels 54 (z. B. flüssiger Stickstoff) eingerichtet. Zum Kühlbetrieb ist zwischen dem Kühlmittelreservoir 51 und dem Kühlmittelbehälter 52 eine Zirkulation des Dampfes des flüssigen Stickstoffs über die Schläuche 53 (siehe Pfeil) vorgesehen. Das Kühlmittelreservoir 51 bildet einen Kryostaten. Im Kühlbehälter 52 wird z. B. eine Temperatur im Bereich von -20°C bis -150°C vorgesehen. Die Komponenten der Kühleinrichtung 50 sind aus an sich bekannten, thermisch isolierenden Materialien aufgebaut.

Figur 3 illustriert einen optional vorgesehenen Regelkreis 60 mit einer Regeleinrichtung 61, die zur Betätigung des Injektorventils 13, des Fluiddruckventils 27 und/oder des Schwingelements 18 in Abhängigkeit von mindestens einem Depositions-Referenzwert eingerichtet ist. Der Depositions-Referenzwert ist zum Beispiel vom Messsignal einer Mikroskopeinrichtung 72 oder einer anderen Abbildungs- oder Überwachungseinrichtung abgeleitet und für einen Betriebszustand der Depositionsvorrichtung 100 charakteristisch. Wenn beispielsweise in einem gegebenen Betriebszustand die Menge und/oder die Eindringtiefe des biologischen Materials 1 im Zielsubstrat 2 nicht den gewünschten Parametern entspricht, kann mit dem Regelkreis 60 mindestens eines der Teile 13, 27 und 18 verstellt werden, um die Zahl der zugeführten Partikel des biologischen Materials 1 zu erhöhen oder zu erniedrigen und/oder um die mit der Antriebseinrichtung 20 ausgeübte Antriebskraft zu erhöhen oder zu erniedrigen.

Figur 3 illustriert als weitere Komponente die Beleuchtungseinrichtung 91, die zur Beleuchtung des Zielsubstrats 2 eingerichtet ist und zum Beispiel eine Weißlichtquelle oder eine Laser-Quelle enthält. Die Beleuchtungseinrichtung 91 und die Mikroskopeinrichtung 72 können mit den übrigen Komponenten der Depositionsvorrichtung 100 fest verbunden und zum Beispiel auf der Außenseite des Kühlbehälters 52 befestigt sein. Anstelle der Mikroskopieeinrichtung 72 kann ein anderes bildgebendes Instrument, wie zum Beispiel eine Ultraschalleinrichtung oder eine Tomographieeinrichtung vorgesehen sein.

Zur Durchführung des erfindungsgemäßen Depositionsverfahrens wird die Depositionsvorrichtung 100 relativ zum Zielsubstrat 2, insbesondere in Bezug auf einen zu behandelnden Wundbereich 2.1 ausgerichtet. Nach Erfassung der Topographie des Wundbereichs 2.1 mit der Mikroskopeinrichtung 72 kann ggf. eine Feinjustierung der Lenkeinrichtung 40 vorgesehen sein. Nach der Justierung wird das biologische Material 1 in Form gefrorener Partikel über den Einführtrichter 14 und das Injektorventil 13 in die Beschleunigungsleitung 22 eingeführt, durch die Luft oder Stickstoff strömt. Hierzu werden die Ventile 13 und 27 geöffnet und das Schwingelement 18 betätigt. Die gefrorenen Partikel des biologischen Materials 1 treten durch die Lenkeinrichtung 40 auf freie Bewegungsbahnen aus, die zum Wundbereich 2.1 führen. In Abhängigkeit von dem beobachteten Depositionsergebnis wird die Zufuhr von biologischem Material fortgesetzt, unterbrochen oder zum Beispiel in Bezug auf den Typ des biologischen Materials modifiziert.

In Figur 4 ist eine abgewandelte Ausführungsform der erfindungsgemäßen Depositionsvorrichtung 100 gezeigt, die sich durch eine kompakte Bauform und manuelle Handhabbarkeit auszeichnet. Zu Illustrationszwecken ist die Depositionsvorrichtung 100 in Figur 4 teilweise in perspektivischer Phantomansicht und teilweise in Schnittansicht gezeigt.

Die Depositionsvorrichtung 100 hat einen langgestreckten, schlauchförmigen Aufbau, der sich von der Antriebseinrichtung 20 über die Ladeeinrichtung 10 mit der Präparationseinrichtung 30 zur Lenkeinrichtung 40 erstreckt. In der Depositionsvorrichtung 100 sind mindestens zwei, sich entlang der Schlauchform erstreckende Kammern vorgesehen, die erstens das beschleunigende Fluid zur Übertragung der Antriebskraft auf das biologische Material und zweitens ein Kühlmittel zur Kühlung des biologischen Materials und dessen Überführung in den gefrorenen Zustand aufnehmen. Die Kammern bilden zum Beispiel koaxial angeordnete Gas- und/oder Flüssigkeitsleitungen.

Die Antriebseinrichtung 20 umfasst einen koaxialen Aufbau mit einer inneren Druckleitung 26 und einer äußeren Kühlmittelleitung 28, die jeweils entsprechend mit einer Druckquelle und einem Kühlmittelreservoir (beide nicht dargestellt) verbunden sind. Beide Leitungen sind mit steuerbaren Ventilen ausgestattet. Die innere Druckleitung 26 besteht zum Beispiel aus einem Kunststoffschlauch, der durch Abstandhalter in der äußeren Kühlmittelleitung 28 positioniert ist. Die äußere Kühlmittelleitung 28 besteht aus einem flexiblen Schlauch, der mit einem isolierenden Material aufgebaut ist, z. B. einem metallischen Balgenschlauch mit einer isolierenden Kunststoffbeschichtung.

Die Ladeeinrichtung 10 umfasst eine Kammer, die zur Durchströmung mit dem zentralen Strom des Druckfluids und dem äußeren Strom des Kühlmittels eingerichtet ist. Als Probeninjektor 11 ist ein Tropfendispenser mit mindestens einer Dispenserdüse 11.1 vorgesehen, die in den zentralen Druckfluidstrom ragt. Bei der dargestellten Ausführungsform weist der Tropfendispenser mehrere Dispenserdüsen 11.1 auf, die als Reihe entsprechend der Strömungsrichtung des Druckfluidstroms (siehe Pfeil) angeordnet sind, so dass orteilhafterweise gleichzeitig eine Vielzahl von Tropfen des biologischen Materials 1 in den Druckfluidstrom injiziert werden können. Der Probeninjektor 11 ragt durch den äußeren Kühlmittelstrom in den zentralen Druckfluidstrom, so dass das biologische Material zunächst in flüssiger Form in den Druckfluidstrom injiziert wird. Der Probeninjektor 11 ist mit der schematisch gezeigten Präparationseinrichtung 30 verbunden, die ein Probenreservoir und eine Pumpe zur Förderung einer Suspension oder Lösung des biologischen Materials zum Probeninjektor 11 enthält.

Stromabwärts von der Ladeeinrichtung 10 erstreckt sich die Beschleunigungsleitung 22, die koaxial von einem zweiten Abschnitt der Kühlmittelleitung 28 umgeben ist, bis zur Lenkeinrichtung 40. Bei der Bewegung des biologischen Materials 1 im Druckfluidstrom durch die Beschleunigungsleitung 22 erfolgt durch einen Wärmeaustausch mit dem Kühlmittelstrom in der Kühlmittelleitung 28 eine Abkühlung des biologischen Materials unter dessen Gefrierpunkt, so dass das biologische Material spätestens beim Durchtritt durch die Lenkeinrichtung 40 im gefrorenen Zustand ist. Die Länge der Beschleunigungsleitung 22 von der Ladeeinrichtung 10 bis zur Lenkeinrichtung 40 beträgt zum Beispiel 4 cm bis 5 m.

Die Lenkeinrichtung 40 umfasst eine Düsenanordnung 41 mit einem Düsenkanal 42, ein Handstück 43, Kühlkammern 44, eine Betätigungseinrichtung 45, 46 und einen Ausströmkanal 47. Das Handstück 43 bildet einen Hohlzylinder aus einem thermisch isolierenden Kunststoffmaterial, in dem die Komponenten 44 bis 47 angeordnet sind und der sich in Stromabwärtsrichtung zur Düsenanordnung 41 konusförmig verjüngt. Die Kühlkammern 44 sind dazu eingerichtet, das durch die Kühlmittelleitung 28 strömende Kühlmittel aufzunehmen und den gefrorenen Zustand des biologischen Materials 1 weiter einzustellen oder zu erhalten. Bei ausreichender thermischer Isolation des Handstücks 43 und ausreichender Länge der Beschleunigungsleitung 22 kann auf die Kühlkammern 44 verzichtet werden.

Die Betätigungseinrichtung umfasst einen Ventilstößel 45, dessen freies Ende aus dem Handstück 43 ragt, und eine Feder 46, mit der der Ventilstößel 45 federnd gelagert ist. Beispielsweise ist vorgesehen, dass im entspannten Zustand die Beschleunigungsleitung 22 geschlossen und vom Düsenkanal 42 getrennt ist. Durch Ausübung eines mechanischen Drucks auf dem Ventilstößel 45, z. B. mit einem Finger, wird eine Durchgangsöffnung im Ventilstößel 45 mit der Beschleunigungsleitung 22 ausgerichtet, so dass die Verbindung zum Düsenkanal 42 freigegeben wird. Vorzugsweise ist die Betätigungseinrichtung 45, 46 zusätzlich mit einem Schalter (nicht dargestellt) ausgestattet, mit dem der Probeninjektor 11 und eines der Ventile in den Leitungen 26, 28 betätigbar sind.

Die Überströmleitung 47 dient der Ableitung von Kühlmittel aus der Kühlmittelleitung 28 und den Kühlkammern 44. Die Überströmleitung 47 ist aus Sicherheitsgründen so angeordnet, dass die Hand einer die Lenkeinrichtung 40 haltenden Person nicht vom austretenden Kühlmittel getroffen wird.

Zur Durchführung des erfindungsgemäßen Depositionsverfahrens strömt gasförmiges oder flüssiges Kühlmittel, z. B. Dampf flüssigen Stickstoffs, mit einer vorbestimmten Kühlmitteltemperatur durch die Kühlmittelleitung 28 von der Antriebseinrichtung 20 über die Ladeeinrichtung 10 bis zur Lenkeinrichtung 40. Mit der Druckfluidleitung wird ein Druckfluidstrom, z. B. aus Stickstoff oder Luft, durch die Ladeeinrichtung 10 und die Beschleunigungsleitung 22 zur Lenkeinrichtung 40 geleitet. Mit dem Probeninjektor 11 werden tropfenförmige Proben biologischen Materials 1 in den Druckgasstrom injiziert und mit diesem stromabwärts durch die Beschleunigungsleitung 22 transportiert. Die Injektion der Tropfen erfolgt durch Gaspulse oder mit einem piezoelektrischen Tropfengeber. Auf dem Weg durch die Beschleunigungsleitung 22 wird das biologische Material 1 in den gefrorenen Zustand überführt. Durch den Düsenkanal 42 erfolgt der Austritt des biologischen Materials 1 auf eine freie Bewegungsbahn hin zu einem Zielsubstrat (nicht dargestellt).

Die Ausführungsform der Depositionsvorrichtung 100 gemäß Figur 4 kann dahingehend modifiziert werden, dass die Funktion der Kühlung des biologischen Materials mit dem Druckgasstrom erfüllt wird. In diesem Fall kann der koaxiale Aufbau durch eine einfache, thermisch isolierte Leitung ersetzt werden. Als Druckgas wird zum Beispiel unter Druck stehender Dampf flüssigen Stickstoffs verwendet. In diesem Fall verfolgt ein Gefrieren der Tropfen des biologischen Materials 1 unmittelbar nach Austritt aus dem Probeninjektor 11. Des Weiteren kann in diesem Fall auf den koaxialen Aufbau der Beschleunigungsleitung 22 mit der Kühlmittelleitung 28 verzichtet werden. Die Kühlmittelleitung 28 kann durch eine evakuierte Kammer ersetzt werden, die die Beschleunigungsleitung 22 umgibt. Des Weiteren kann die Düsenanordnung 41 mit einem einzelnen Düsenkanal 42 durch einen Aufbau mit mehreren Düsenkanälen ersetzt werden, wie er zum Beispiel in Figur 3 gezeigt ist. Die Depositionsvorrichtung 100 gemäß Figur 4 kann mit weiteren Komponenten, z. B. zur Überwachung der Depositionsvorrichtung und/oder zur Abbildung des Zielsubstrats ausgestattet sein.

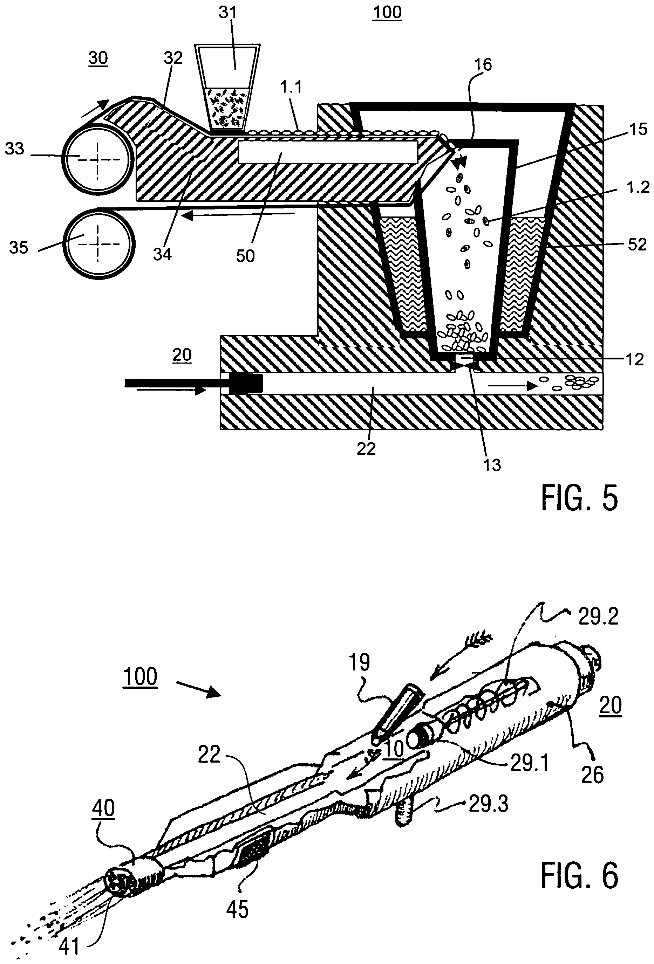

Figur 5 zeigt eine weitere Depositionsvorrichtung 100 (keine Ausführungsform der Erfindung) in schematischer Schnittansicht. Die Depositionsvorrichtung 100 weist eine Präparationseinrichtung 30 auf, mit welcher die zu verschießenden gefrorenen Partikel 1 direkt vor dem Verschießen aus einer Zellsuspension erzeugt werden. Von einem Vorratsgefäß 31 aus gelangt die Zellsuspension auf ein darunter vorbeilaufendes Förderband 32. Das Förderband 32 läuft von einer Eingangstrommel 33 aus über einen Körper 34 unter dem Vorratsgefäß 31 durch und nimmt dabei Zellsuspension auf. Das Förderband 32 ist vorzugsweise mindestens an der Oberfläche oder insgesamt hydrophob, so dass die Zellsuspension darauf einzelne Tropfen 1.1 bildet. Von dem Vorratsgefäß 31 aus werden die Tropfen 1.1 dann über den Körper 34, welcher beispielsweise aus PTFE besteht, und über eine Kühleinrichtung 50 in Form einer Kühlplatte zur Öffnung 16 eines Vorratsbehälters 15 transportiert. Dabei werden die Tropfen 1.1 auf Temperaturen unter - 30 °C gefroren und bilden somit gefrorene Partikel 1.2. In der Öffnung 16 des Vorratsbehälters läuft das Förderband 32 über eine Kante, wobei es stark deformiert wird. Durch die dabei auftretende Biegespannung platzen die gefrorenen Partikel 1.2 von dem Förderband 32 ab und gelangen so in den Vorratsbehälter 15 für die gefrorenen Partikel 1.2.

Der Vorratsbehälter 15 ist von einem mit Kühlmittel gefüllten Kühlmittelbehälter 52 umgeben. Als Kühlmittel kommt beispielsweise flüssiger Stickstoff zum Einsatz. Das gesamte System ist vorzugsweise thermisch isoliert. Von dem Vorratsbehälter 15 gelangen die gefrorenen Partikel (Kristalle) 1.2 zum Beispiel durch Gravitation in eine Injektorleitung 12. Durch die Injektorleitung und ein verschließbares Injektorventil 13 gelangen die Partikel 1.2 schließlich zu einer Antriebseinrichtung 20, von wo aus sie direkt verschossen werden können. Vorzugsweise gelangen sie direkt durch das Injektorventil in eine Beschleunigungsleitung 22 der Antriebseinrichtung 20.

Über die Drehung einer Aufnahmerolle 35 kann die Geschwindigkeit des Förderbandes 32 gesteuert werden. Mit einer zusätzlichen Steuerung der Kühlplatte 50 ist so eine Kontrolle des Gefriervorganges möglich, so dass gezielt Temperaturprogramme gefahren werden können. Dies ermöglicht die Verwendung der für die Kryokonservierung der jeweiligen Zellen oder anderen biologischen Materialien optimalen Temperaturprogramme. Zudem kann mit dieser Anordnung die Menge der zu verschießenden Partikel frei gewählt werden.

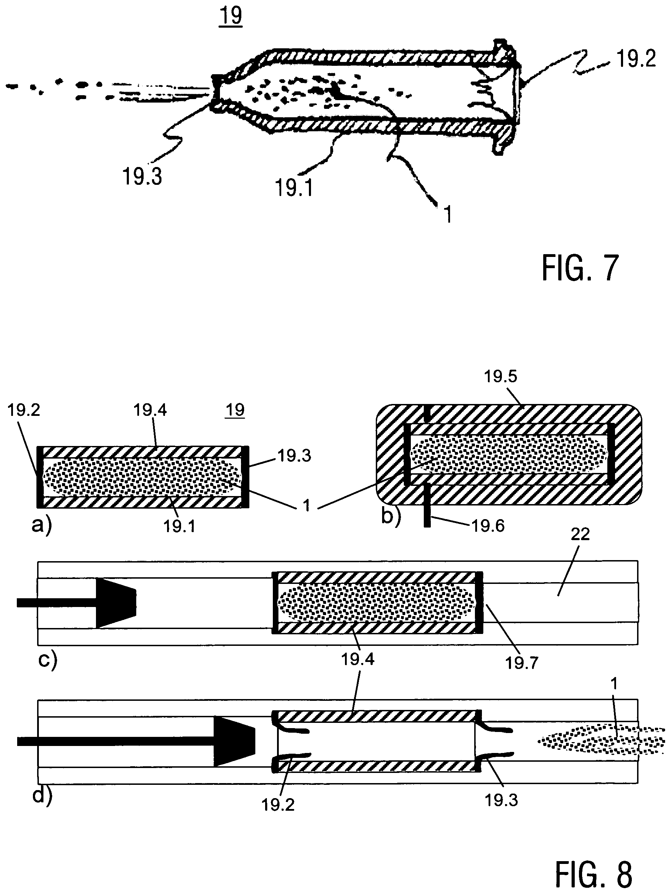

Die Figuren 6 und 7 illustrieren eine weitere Depositionsvorrichtung 100 (keine Ausführungsform der Erfindung), bei der die Ladeeinrichtung 10 mit einer Injektorpatrone 19 vorgesehen ist. Bei dieser Depositionsvorrichtung 100 umfasst die Antriebseinrichtung 20 ein langgestrecktes Gehäuse, dessen hinteren und vorderen Teile entsprechend die Druckleitung 26 und die Beschleunigungsleitung 22 bilden und an dessen stromabwärts gelegenen, vorderen Ende die Lenkeinrichtung 40 mit der Düseneinrichtung 41 angeordnet ist.

Die Ladeeinrichtung 10 umfasst eine Kammer zwischen den Druck- und Beschleunigungsleitungen 26, 22. Diese Kammer ist zur Aufnahme der Injektorpatrone 19 eingerichtet. Die Injektorpatrone 19 umfasst eine Patronenhülse 19.1 aus einem thermisch isolierenden Kunststoff (Figur 7). Die Patronenhülse 19.1 weist in Längsrichtung eine Einströmöffnung und eine Ausströmöffnung auf, die entsprechend mit Abdeckelementen 19.2, 19.3 verschlossen sind. Mit den Abdeckelementen 19.2, 19.3 ist das Innere der Patronenhülse 19.1 während deren Lagerung verschlossen. Bei Beaufschlagung der Abdeckelemente 19.2, 19.3 mit einem Fluiddruck der Antriebseinrichtung 20 werden die Abdeckelemente 19.2, 19.3 geöffnet, so dass der Druckfluidstrom durch die Injektorpatrone 19 hindurchtreten und das biologische Material 1 durch die Beschleunigungsleitung 22 und die Lenkeinrichtung 40 zum Zielgewebe (nicht dargestellt) transportieren kann.

Die Injektorpatrone 19 enthält das biologische Material 1 im gefrorenen Zustand. Vorteilhafterweise kann die Injektorpatrone 19 mit dem gefrorenen Material 1 unabhängig vom Betrieb der Depositionsvorrichtung 100 gefüllt, gelagert und transportiert werden.

Die Injektorpatrone 19 wird in der Ladeeinrichtung 10 durch eine Spanneinrichtung gehalten, die in der Druckfluidleitung 26 angeordnet ist und einen Haltering 29.1, eine Spannfeder 29.2 und einen Spannhebel 29.3 umfasst. Zum Einsetzen der Injektorpatrone 19 durch eine seitliche Öffnung des Gehäuses der Depositionseinrichtung 100 wird der Haltering 29.1 mit dem Spannhebel 29.3 zurückgezogen und nach Einlegen der Injektorpatrone 19 freigegeben. Der Haltering 29.1 sitzt auf dem rückseitigen Rand der Patronenhülse 19.1 auf und lässt das Abdeckelement 19.2 zur Beaufschlagung mit dem Fluiddruck der Antriebseinrichtung 20 frei.

Zur Durchführung des Depositionsverfahrens wird eine befüllte, gekühlte Injektorpatrone 19 in die Ladeeinrichtung 10 eingesetzt. Anschließend wird die Depositionsvorrichtung 100 relativ zum Zielsubstrat positioniert. Mit einer Betätigungstaste 45 wird ein Druckfluidventil in der Druckleitung 26 geöffnet, so dass das Druckfluid mit hoher Geschwindigkeit durch die Druckleitung 26 strömt, die Abdeckelemente 19.2, 19.3 der Injektorpatrone 19 durchbricht und das biologische Material durch die Beschleunigungsleitung 22 mitreißt.

Figuren 8a bis 8d zeigen eine alternative Variante einer Injektorpatrone 19 und deren Verwendung. Figur 8a zeigt die Patrone 19 in Schnittansicht mit einer thermisch isolierenden Hülle 19.4, die innen durch eine druckbelastete Hülse 19.1 den Innenraum mit dem gefrorenen Partikeln 1 umschließt. Die Hülse 19.1 weist eine Einströmöffnung und eine Ausströmöffnung auf, die beide jeweils mit einer dünnen Membran 19.2, 19.3 beispielsweise aus Plastik, Zellulose oder Metall verschlossen sind. Für eine Lagerung oder einen Transport befindet sich die Injektorpatrone 19 in einer zusätzlichen thermisch isolierenden Transporthülle 19.5. Die Transporthülle 19.5 verfügt über ein Aufreißband 19.6, bei dessen Abreißen sie sich in zwei Teile trennen lässt, die dann von der Injektorpatrone beispielsweise nach vorn und nach hinten abgenommen werden können (Figur 8b).

Für die Verwendung in einer Depositionsvorrichtung wird die Injektorpatrone 19 ohne Transporthülle 19.5 in eine Patronenaufnahme in der Beschleunigungsleitung 22 (siehe z.B. Figur 6) eingesetzt. Die Position der Injektorpatrone 19 in der Beschleunigungsleitung 22 ist dabei so gesichert, dass sie bei einer Druckerzeugung von der Einströmseite her nicht in Richtung der Ausströmseite verschoben werden kann. Dies wird beispielsweise damit erreicht, dass der Innendurchmesser der Beschleunigungsleitung 22 auf der Ausströmseite 19.7 der Injektorpatrone 19 kleiner ist als der Außendurchmesser der Hülle 19.4 (Figur 8c). Zum Verschießen der gefrorenen Partikel 1 werden die Membranen 19.2 und 19.3 durch einen kurzen starken Druckanstieg oder mechanisch durchschlagen. Die gefrorenen Partikel 1 verlassen dann die Patrone 19 und die Beschleunigungsleitung 22 in Richtung der Ausströmöffnung (nach rechts in Figur 8d).

Alternativ kann die Hülse 19.1 an der Einströmseite mit einer deformierbaren Membran 19.2 verschlossen sein, die durch den Schießvorgang nicht durchtrennt wird sondern nur den Impuls weitergibt. In diesem Fall wird nur die Membran 19.3 auf der Ausströmseite durchbrochen.

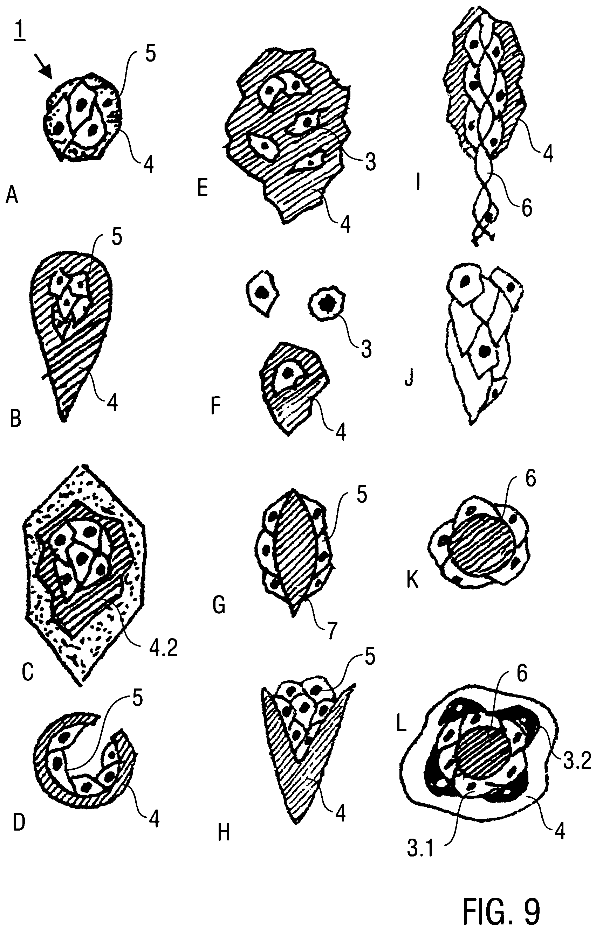

Die Figuren 9A bis 9L zeigen verschiedene Varianten der Form und Zusammensetzung des biologischen Materials 1, das erfindungsgemäß im gefrorenen Zustand in das Zielsubstrat eingebettet wird, in vergrößerten, schematischen Querschnittsansichten. Die Querschnittsdimension der gefrorenen Partikel des biologischen Materials ist typischerweise im Bereich von 3 µm bis 5 mm, vorzugsweise im Bereich von 20 µm bis 5 mm gewählt. Die Partikel haben typischerweise ein Volumen im Bereich von 25 µm³ und 20 mm³. Vorzugsweise umfasst das biologische Material 1 mindestens eine biologische Zelle 3 oder Zellgruppe 5 zum Beispiel von eukaryotischen Zellen und mindestens eine Zusatzsubstanz 4. Die Zusatzsubstanz 4 ist typischerweise das Umgebungsmedium, in dem die Zelle für die Deposition vorbereitet (präpariert) wurde. Alternativ kann das biologische Material ausschließlich aus der im gefrorenen Zustand zugeführten Zusatzsubstanz, wie zum Beispiel einem Füllstoff, einer Suspensionsflüssigkeit, oder einem Kultivierungsmedium bestehen.

Gemäß Figur 9A umfasst das biologische Material eine Zusammensetzung mit der Zellgruppe 5 und der Zusatzsubstanz 4. Die Zusatzsubstanz 4 weist ein im Vergleich zur Zellgruppe 5 vernachlässigbares Volumen auf. Die Reduzierung der Zusatzsubstanz 4 auf ein Minimum (gefrorener Rest einer Umgebungslösung) ist von Vorteil, wenn die Zellen mit minimaler Verdrängung und geringster Kontamination in das Zielsubstrat eingebracht werden sollen, wie es insbesondere bei Anwendungen in der Medizin und beim Tissue Engineering von Interesse ist.

Alternativ kann gemäß Figur 9B das biologische Material die Zellgruppe 5 und die Zusatzsubstanz 4 umfassen, deren Volumen gleich oder größer als das Volumen der Zellgruppe 5 ist. Diese Variante hat den Vorteil, dass eine definierte Menge einer vorbestimmten Lösung, die zum Beispiel Wirkstoffe enthalten kann, mit den Zellen in das Zielsubstrat eingebracht werden kann. Des Weiteren bietet die Umhüllung mit der gefrorenen Zusatzsubstanz 4 einen verbesserten Schutz bei der Einbettung der Zellgruppe 5 im Zielsubstrat. Schließlich vereinfacht ein großes Volumen der Zusatzsubstanz 4 die Möglichkeit, die gefrorenen Partikel des biologischen Materials mit einer vorbestimmten Form zu bilden. Es kann beispielsweise beim Einfrieren eine Partikelform eines langgestreckten Ellipsoiden gebildet werden, die das Eindringen in das Zielsubstrat erleichtert. Des Weiteren kann durch die Formgebung die Lage des Partikels im Zielsubstrat beeinflusst werden.

Das biologische Material kann erfindungsgemäß mehrere Zusatzsubstanzen 4.1, 4.2 enthalten, wie beispielhaft in Figur 9C gezeigt ist. Die Zellgruppe 5 wird von mehreren Schichten umhüllender Lösungen im gefrorenen Zustand umgeben. Beispielsweise enthält die innere Zusatzsubstanz 4.1 ein Kultivierungsmedium mit einem Kryoprotektivum und die äußere Zusatzsubstanz 4.2 ein Kultivierungsmedium ohne Kryoprotektivum. Ein derartiger mehrschichtiger Aufbau kann erzeugt werden, indem zum Beispiel zunächst die Zellgruppe mit der inneren Zusatzsubstanz eingefroren und anschließend durch Eintauchen mit der äußeren Zusatzsubstanz beschichtet wird. Der Vorteil dieser Ausführungsform der Erfindung ergibt sich beim Auftauen des biologischen Materials im Zielsubstrat. Beim Auftauen vermischen sich beide Zusatzsubstanzen 4.1, 4.2, so dass die Konzentration des Kryoprotektivums im Gesamtvolumen beider Zusatzsubstanzen sinkt. Vorteilhafterweise wird damit eine erhöhte Überlebensrate der eingebetteten Zellen durch einen verringerten Stress beim Auftauen erreicht.

Die Zusatzsubstanz 4 kann gemäß einer weiteren Variante (Figur 9D) einen Hohlkörper, insbesondere eine Hohlkugel bilden. In diesem Fall kann von Vorteil sein, dass die Zellgruppe 5 eine freie Zelloberfläche aufweist und dennoch bei der Einbettung in das Zielsubstrat durch die Zusatzsubstanz 4 geschützt ist. Der Hohlkörper der Zusatzsubstanz 4 gemäß Figur 9D kann durch eine gefrorene Flüssigkeit oder ein gefrorenes Gel oder gemäß einer weiteren Variante der Erfindung durch ein festes Trägermaterial (siehe auch Figur 9G) gebildet werden.

Alternativ kann das biologische Material eine Zusammensetzung aus einer Vielzahl einzelner Zellen 3 und der Zusatzsubstanz 4 gebildet werden (siehe Figur 9E). Von Vorteil bei Anwendungen im Tissue Engineering oder beim Klonieren von Zellen ist es, wenn gemäß Figur 9F einzelne Zellen 3 ohne oder mit Zusatzsubstanz 4 im Zielsubstrat eingebettet werden.

Gemäß Figur 9G kann die Zusatzsubstanz, z. B. als gefrorene Flüssigkeit oder gefrorenes Gel (oder alternativ als festes Trägermaterial 6) einen Träger für die Zellgruppe 5 bilden. In diesem Fall wird vorteilhafterweise eine große mechanische Stabilität des gefrorenen Partikels und ein direkter Kontakt der Zellen 5 mit dem Zielsubstrat erreicht.

Figur 9H zeigt eine weitere Ausführungsform einer erfindungsgemäß verwendeten Zusammensetzung einer Zellgruppe 5 mit einer Zusatzsubstanz 4, die einen schützenden Hohlraum für die Zellgruppe 5 bildet.

Eine weitere Variante einer Zusammensetzung mit einem festen Trägermaterial 6 ist in Figur 9I gezeigt. Das Trägermaterial 6 umfasst ein Substrat, auf dem biologische Zellen 3 aufgewachsen oder adheriert sind. Das Substrat umfasst zum Beispiel ein Gewebe, einen Faden, ein Netz, ein Gel, ein Polymer oder dergleichen. Vorteilhafterweise bildet das feste Trägermaterial 6, ggf. in Verbindung mit der Zusatzsubstanz 4 im gefrorenen Zustand einen Festkörper, der wie die anderen illustrierten gefrorenen Partikel in das Zielsubstrat eingeschossen werden kann. Abweichend von der in Figur 9I gezeigten Variante kann das biologische Material ohne die Zusatzsubstanz 4, d.h. ausschließlich mit biologischen Zellen 3 und dem festen Trägermaterial 6 gebildet sein.

Erfindungsgemäß kann das biologische Material ein Konglomerat (Zusammenballung, Gemisch) aus verschiedenen gefrorenen Teilen sein, die Zellen enthalten oder nicht. Ein Beispiel eines derartigen Konglomeratpartikels ist schematisch in Figur 9J gezeigt. Konglomerate haben den Vorteil, dass die einzelnen Teile nach Bedarf zusammengesetzt werden können und nach dem Auftreffen auf das Zielsubstrat oder nach dem Auftauen zerfallen und getrennt wirksam werden.

Ein weiteres Beispiel für ein biologisches Material mit einem festen Trägermaterial 6 ist in Figur 9K gezeigt. In diesem Fall bildet das feste Trägermaterial 6 einen Kernkörper, der zum Beispiel Substanzen beinhalten kann, die erst nach dem Einbetten und Auftauen des biologischen Materials freigesetzt werden. Vorteilhafterweise wird damit der Wirkungsbereich der Substanzen auf den Depositionsort begrenzt. Das feste Trägermaterial 6 kann ein kugelförmiger Festkörper (Bead), ein Gel, ein Polymer oder ein gefrorener Flüssigkeitspartikel sein.

Die oben erläuterten Varianten der Zusammensetzung des biologischen Materials können kombiniert und erweitert werden, wie beispielhaft in Figur 9L illustriert ist. Es können beispielsweise mehrere Zelltypen 3.1, 3.2 auf einem zentralen Trägermaterial 6 angeordnet und mit einer äußeren Zusatzsubstanz 4 umhüllt sein. Vorteilhafterweise können so vorgefertigte Gewebepartikel, z. B. mit Alginat umhüllte Inselzellen, in Form von Xenontransplantaten oder für den allogenen Transfer in das Zielsubstrat eingebettet werden.

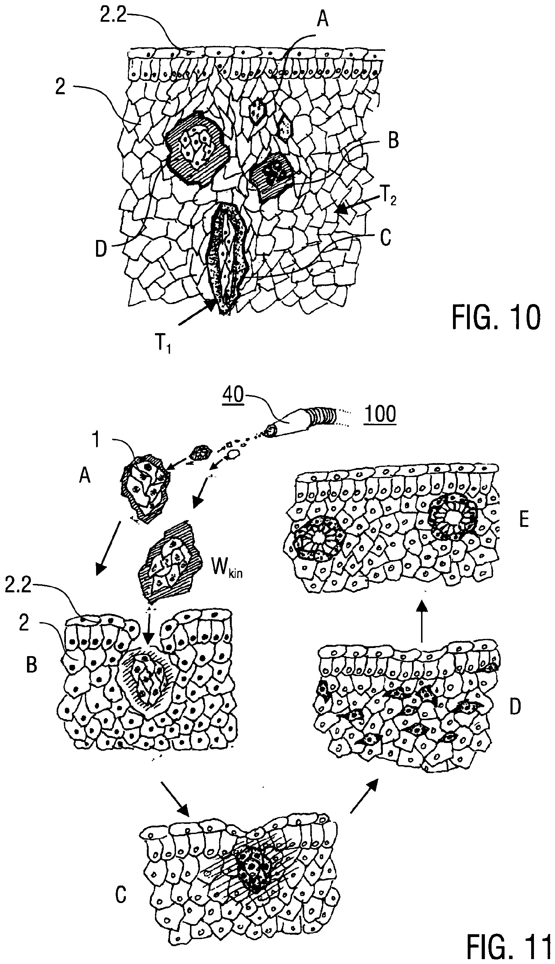

Figur 10 illustriert in schematischer Schnittansicht verschiedene Typen eingebetteter Partikel gefrorenen Materials in einem Zielsubstrat 2, das durch ein Gewebe biologischer Zellen gebildet wird. Die Partikel A, B, C und D werden im gefrorenen Zustand mit einer ersten Temperatur T₁ in das Zielsubstrat 2 eingebettet, das eine Umgebungstemperatur T₂ aufweist. Die Temperatur T₁ kann in Abhängigkeit von den verwendeten Materialien und der konkreten Anwendung der Erfindung im Bereich zwischen zum Beispiel -270°C bis zum Gefrierpunkt des Partikels, vorzugsweise im Bereich von -40°C bis - 160°C gewählt sein. Vorteilhaft sind insbesondere Temperaturen unterhalb von -130°C, da in diesem Temperaturbereich zum Beispiel CO₂ stabil ist und Wassereiskristalle kein migratorisches Wachstum zeigen. Die Temperatur T₁ kann des Weiteren gewählt werden, um eine vorbestimmte Eindringtiefe im Zielsubstrat 2 zu erreichen. Da beim Einbetten des biologischen Materials im Zielsubstrat 2 durch Reibungswärme und Wärmeaustausch mit dem Zielsubstrat 2 eine Erwärmung der Partikel erfolgt, kann mit der Wahl der Temperatur T₁ unter Berücksichtigung der Größe der gefrorenen Partikel die Eindringtiefe des biologischen Materials 1 im Zielsubstrat 2 eingestellt werden. Die Umgebungstemperatur T₂ ist typischerweise die Raumtemperatur, eine Kultivierungstemperatur oder die Gewebetemperatur des lebenden Organismus (z. B. 36°C bis 37°C beim Menschen).

Figur 10 zeigt, dass durch die Form und Größe der Partikel Depositionsparameter, wie zum Beispiel die Eindringtiefe unter die Oberfläche 2.2 des Zielsubstrats 2, die Verdrängung im Zielsubstrat 2 (z. B. Verdrängung von Zellen in einem Zielgewebe) und die Ausrichtung des eingebetteten biologischen Materials beeinflusst werden können. Die Partikel tauen unmittelbar nach dem Eindringen in das Zielsubstrat 2, während der Bewegung in diesem oder nach Erreichen der Depositionsposition auf. Damit sind Eindringtiefen im Bereich von einigen µm bis hin zu einigen mm oder sogar cm erreichbar, wobei Verletzungen oder Verdrängungen im Volumen des Zielsubstrats 2 minimiert werden.

Ein wesentlicher Vorteil des erfindungsgemäßen Verfahrens besteht darin, dass die Einbettung biologischen Materials nicht auf Zellen oder Zellgruppen beschränkt ist, sondern entsprechend auch gefrorene Lösungen, insbesondere gefrorene Wirkstoffe, oder bei Raumtemperatur weiche oder elastische Materialien, wie zum Beispiel Gele, Matrixmaterialien oder Polymere durch Einfrieren in eine starre Form gebracht und entsprechend geschützt und stabil in das Zielsubstrat eingebettet werden können.

Figur 11 illustriert in schematischer Schnittansicht verschiedene Phasen der erfindungsgemäßen Deposition biologischen Materials 1 in ein Zielsubstrat 2 und anschließende Entwicklungsschritte, die nach der Einbettung mit dem biologischen Material 1 im Zielsubstrat 2 erhalten werden können. Bei Schritt A wird das biologische Material 1 im gefrorenen Zustand auf einer freien Bewegungsbahn von der Lenkeinrichtung 40 der Depositionsvorrichtung 100 (perspektivische Teildarstellung) zur Oberfläche 2.2 des Zielsubstrats 2 bewegt. Durch die Beaufschlagung mit einer Antriebskraft weist das biologische Material 1 beim Eintreffen an der Oberfläche 2.2 eine vorbestimmte kinetische Energie Wₖᵢₙ auf, die das Eindringen in das Zielsubstrat 2 (z. B. ein biologisches Gewebe) ermöglicht. Bei Schritt B erfolgt das Eindringen des gefrorenen biologischen Materials 1 in das Zielsubstrat 2. Beim Eindringen verdrängt oder zerstört das biologische Material 1 minimale Teile des Zielsubstrats 2. Vorteilhafterweise könnten jedoch aufgrund der geringen Partikelgröße Verdrängungsräume, wie zum Beispiel ein vom Partikel gebildeter Einschusskanal oder Verletzungen schnell geschlossen und geheilt werden. Gleichzeitig erfolgt durch den unmittelbaren Kontakt des biologischen Materials 1 mit dem Zielsubstrat 2 ein Wärmeaustausch, bis das biologische Material 1 aufgetaut ist (Schritt C). Dabei diffundiert die im biologischen Material 1 ggf. enthaltene Zusatzsubstanz (Kultivierungsmedium, Wirkstoff oder Wachstumsfaktor) in die Umgebung des Depositionsortes.

Die Schritte D und E illustrieren Entwicklungsschritte, die optional bei der Einbettung von zum Beispiel Stammzellen oder Vorläuferzellen in ein biologisches Gewebe auftreten können. Einzelne differenzierte Zellen können durch Auflösung und Wandern der ursprünglich eingebetteten Zellgruppe und/oder durch Differenzierung vorhandener Zellen in verteilter Form im Gewebe auftreten (Schritt D). Gleichzeitig oder anschließend können sich ausgehend von den vereinzelten Zellen physiologische Gewebeformen ausbilden. Beispielsweise ist bei Schritt E eine beginnende Angiogenese illustriert.

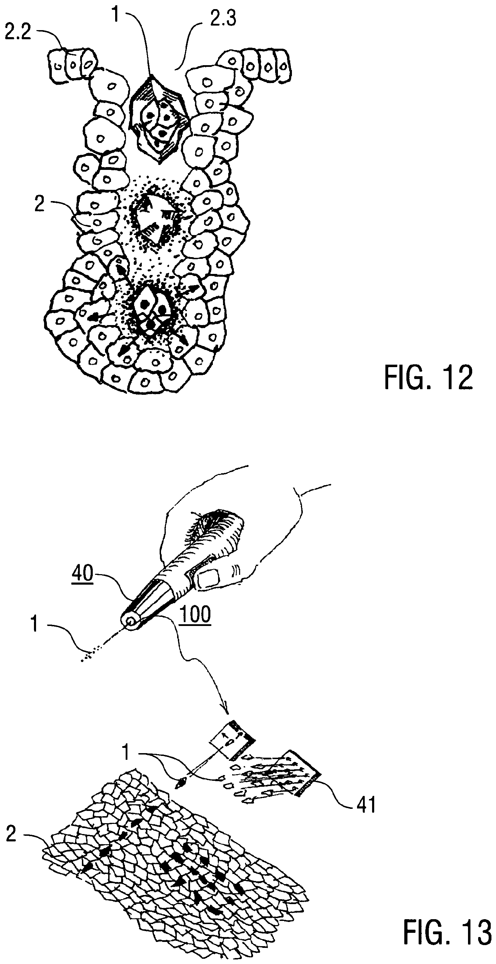

Die in Schritt A der Figur 11 gezeigte freie Flugbahn des biologischen Materials 1 muss nicht zwingend zwischen der Lenkeinrichtung 40 und der Oberfläche 2.2 des Zielsubstrats 2 gegeben sein, sondern kann bei einer abgewandelten Ausführungsform der Erfindung in einem Hohlraum 2.3 des Zielsubstrats 2 gebildet werden (Figur 12). In diesem Fall umfasst die Lenkeinrichtung der Depositionsvorrichtung (nicht dargestellt) einen langgestreckten Düsenkanal, der eine Hohlleitung, wie zum Beispiel eine Kanüle, Spritzenspitze oder Kapillare bildet. Die Lenkeinrichtung kann in das Zielsubstrat 2 bis in den Hohlraum 2.3, wie zum Beispiel ein Hohlorgan, eingeführt werden, um von dort das biologische Material 1 in das umgebende Zielsubstrat 2 einzuschießen.

In Figur 12 ist auch eine weitere Variante des erfindungsgemäßen Verfahrens illustriert, bei der aufeinanderfolgend Partikel mit verschiedenen biologischen Materialien an einem gemeinsamen Depositionsort im Zielgewebe 2 eingebettet werden. Vorteilhafterweise können damit beim Auftauen am Depositionsort bestimmte physiologische Zusammensetzungen, z. B. aus Zellen, Zellbestandteilen, biologischen Makromolekülen, Wirkstoffen und/oder biokompatiblen Zusatzstoffen, z. B. Füllstoffen bereitgestellt werden. Beispielsweise umfasst der in Figur 12 gezeigte unterste Partikel eine Gruppe von Stammzellen, der mit dem mittleren Partikel ein Kultivierungsmedium zugesetzt wird, das zum Beispiel Differenzierungsfaktoren enthält. Vorteilhafterweise kann das Kultivierungsmedium ohne Kryoprotektivum zugeführt werden. Dies ermöglicht ein schnelles Verdünnen des im untersten Partikel enthaltenen Kryoprotektivums und damit eine erhöhte Vitalitätsrate der eingebetteten Zellen. Schließlich kann mit dem obersten Partikel ein weiterer Zelltyp mit einer Kultivierungslösung eingebracht werden, mit dem die Differenzierung der Stammzellen beeinflusst werden soll.

Figur 13 illustriert eine weitere Anwendung der Erfindung bei der Deposition biologischen Materials 1 in ein Zielsubstrat 2, das durch eine Zellkultur, z. B. in einem Kultivierungsgefäß gebildet wird. Mit der Depositionsvorrichtung 100, die als manuell handhabbares Werkzeug gezeigt ist, wird das biologische Material 1 in Abhängigkeit von der Gestalt der Düsenanordnung 41 der Lenkeinrichtung 40 und der Bewegung der Depositionsvorrichtung 100 über dem Zielsubstrat 2 in diesem eingebettet. Beispielhaft ist bei A eine Einzeldüse gezeigt, die für ein serielles Einschießen des biologischen Materials 1 in das Zielsubstrat 2 eingerichtet ist. Des Weiteren ist bei B eine Düsenanordnung 41 mit einer Matrixform der Austrittsdüsen gezeigt, die zum gleichzeitigen Einschießen oder für eine schnelle Musterbildung im Zielsubstrat 2 eingerichtet ist (Formationsbildung). Die gleichzeitige (parallele) Einbettung von biologischem Material 1 im Zielsubstrat 2 ist auch für die Einbettung von Stammzellen in geschädigtes Gewebe von Vorteil, wie es in der regenerativen Medizin angewendet wird.

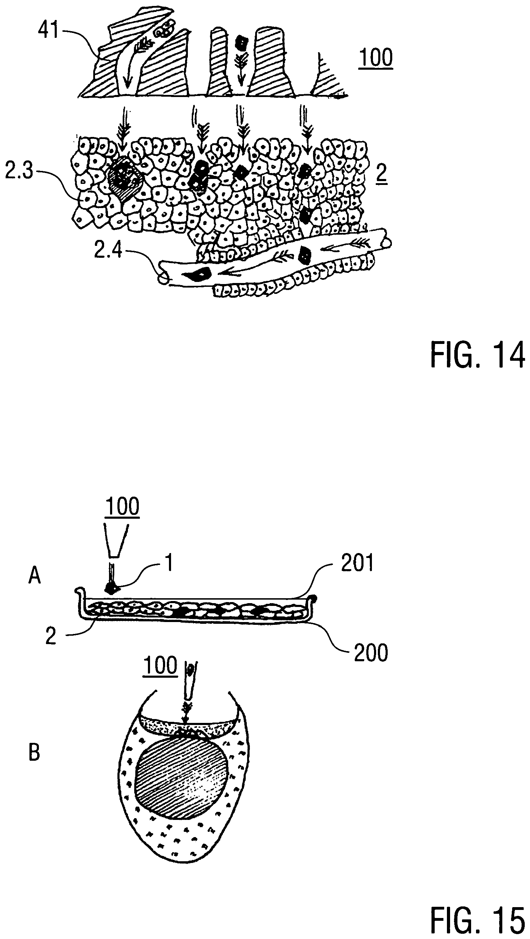

Figur 14 illustriert die Einbettung biologischen Materials in ein inhomogenes Zielsubstrat 2 mit einer Gewebeschicht 2.4 und einem Blutgefäß 2.5. Mit der Düsenanordnung 41 der Depositionsvorrichtung 100 (Teildarstellung) können mehrere Proben des biologischen Materials in das Zielsubstrat 2 eingebettet werden. Bei der Einbettung von Zellen, welche die Eigenschaft haben, im biologischen Gewebe aktiv zu wandern, wie zum Beispiel Stammzellen, Fibroblasten oder Makrophagen, können diese Zellen in das Blutgefäß 2.5 des behandelten Organismus übertreten. Die Position und Größe der eingebetteten Proben des biologischen Materials 1 können in Abhängigkeit von Betriebsparametern der Depositionsvorrichtung 100 und insbesondere der Gestalt der Düsenanordnung 41 gewählt werden.

Weitere Beispiele für die Einbettung biologischen Materials 1 in Zellkulturen sind in den Figuren 15A und 15B gezeigt. Gemäß Figur 15A umfasst das Zielsubstrat 2 eine Zellkultur in einem Kultivierungsgefäß 200. Die Zellkultur umfasst zum Beispiel tierische oder pflanzliche Zellen, Bakterien, Pilze oder Gemische aus diesen. Die Zellkultur kann vorteilhafterweise mit einer Flüssigkeit 201 überschichtet sein, ohne dass die erfindungsgemäße Deposition des biologischen Materials 1 beeinträchtigt wird. Dies ist auch bei der Einbettung in natürliche Kultivierungseinrichtungen (Figur 15B) von Vorteil, wenn das Zielsubstrat 2 durch ein inhomogenes System mit einer Phasengrenze gebildet wird. In Figur 15B ist beispielhaft die Einbettung gefrorener Partikel in ein Vogel- oder Fischei schematisch illustriert. Anwendungen dieser Technik sind bei der gemeinsamen Kultivierung embryonaler Zellen oder anderer Zellen oder Gewebeteile gegeben. Der Vorteil der Erfindung besteht bei dieser Anwendung in der hohen Depositionsgenauigkeit und dem mechanischen Schutz der Zellen bis zum Auftauen. Abweichend von der illustrierten Variante kann die Deposition des biologischen Materials auch in anderen Teilen der natürlichen Kultivierungseinrichtung, z. B. im Eigelb erfolgen.

Weitere Anwendungsbeispiele der Erfindung sind außerdem das Einbringen tiefgefrorener Haarwurzelzellen oder -aggregaten in die Haut von Patienten, sowie das Einbringen von Pigmentzellen oder Stammzellen in vitro und in vivo.

Die in der vorstehenden Beschreibung, den Zeichnungen und den Ansprüchen offenbarten Merkmale der Erfindung können sowohl einzeln als in Kombination für die Verwirklichung der Erfindung in ihren verschiedenen Ausgestaltungen von Bedeutung sein.

## Patentansprüche

1. Depositionsvorrichtung (100), die zur Deposition von biologischem Material (1) in einem gefrorenen Zustand in einem Zielsubstrat (2), welches biologische Zellen enthält, eingerichtet ist, umfassend eine Ladeeinrichtung (10) und eine Antriebseinrichtung (20), wobei
- die Ladeeinrichtung (10) zur Bereitstellung des biologischen Materials (1) an der Antriebseinrichtung (20) eingerichtet ist und die Antriebseinrichtung (20) zur Ausübung einer Antriebskraft auf das biologische Material (1) eingerichtet ist,
- die Antriebseinrichtung (20) zur Ausübung der Antriebskraft derart gebildet ist, dass das biologische Material (1) durch die Wirkung der Antriebskraft eine kinetische Energie erhält, so dass es in das Zielsubstrat (2) unter Verdrängung oder Zerstörung von im Zielsubstrat vorhandenen Zellen eindringen kann, wobei die kinetische Energie gleich einer Verdrängungsarbeit ist, die das biologische Material (1) bis zum Erreichen einer gewünschten Tiefe im Zielsubstrat (2) verrichtet, und
- die Antriebseinrichtung (20) einen Fluidantrieb mit einem Gas umfasst, der zur Erzeugung der Antriebskraft durch Ausübung eines Gasdrucks auf das biologische Material eingerichtet ist,
**gekennzeichnet durch**
- eine Kühleinrichtung (50), die zur Kühlung des biologischen Materials (1) und mindestens eines Teils der Antriebseinrichtung (20) eingerichtet ist, wobei
- die Kühleinrichtung (50) zur Bereitstellung des Gases zur Übertragung der Antriebskraft bei einer Temperatur unterhalb des Gefrierpunkts des biologischen Materials eingerichtet sein.

2. Depositionsvorrichtung gemäß Anspruch 1, die umfasst:
- eine Präparationseinrichtung (30), die zur Bereitstellung des biologischen Materials an der Ladeeinrichtung (10) eingerichtet ist.

3. Depositionsvorrichtung gemäß Anspruch 2, bei der die Präparationseinrichtung (30) einen Tropfengenerator, der zur Erzeugung von Tropfen des biologischen Materials eingerichtet ist, oder einen Partikelgenerator enthält, der zur Erzeugung von gefrorenen Tropfen des biologischen Materials eingerichtet ist.

4. Depositionsvorrichtung gemäß mindestens einem der vorhergehenden Ansprüche, die umfasst:
- eine Lenkeinrichtung (40), die zur Ausrichtung einer Bewegung des biologischen Materials zu dem Zielsubstrat (2) eingerichtet ist.

5. Depositionsvorrichtung gemäß Anspruch 4, bei der die Lenkeinrichtung (40) eine Düseneinrichtung (41) und/oder ein manuell handhabbares Werkzeug mit mindestens einer Hohlleitung (42) aufweist.

6. Depositionsvorrichtung gemäß mindestens einem der vorhergehenden Ansprüche, bei der die Ladeeinrichtung (10) einen Probeninjektor (11) umfasst, der zur Einführung des biologischen Materials (1) in eine Beschleunigungsleitung (22) der Antriebseinrichtung (20) eingerichtet ist.

7. Depositionsvorrichtung gemäß Anspruch 6, bei welcher der Probeninjektor (11) entweder einen Tropfendispenser (13), der zur Zuführung von Tropfen des biologischen Materials in die Beschleunigungsleitung (21) eingerichtet ist, oder einen Partikeldispenser, der zur Zuführung von Partikeln des gefrorenen biologischen Materials in die Beschleunigungsleitung (21) eingerichtet ist, aufweist.

8. Depositionsvorrichtung gemäß Anspruch 7, bei welcher der Probeninjektor (11) eine Injektorpatrone (19) aufweist, die in der Beschleunigungsleitung (21) austauschbar angeordnet ist.

9. Depositionsvorrichtung gemäß mindestens einem der vorhergehenden Ansprüche, bei der die Kühleinrichtung (50) zur Kühlung mindestens eines Teils der Ladeeinrichtung (10) und/oder mindestens eines Teils der Lenkeinrichtung (40) eingerichtet ist.

10. Depositionsvorrichtung gemäß mindestens einem der vorhergehenden Ansprüche, die umfasst:
- einen Regelkreis (60), der mit der Steuerung der Ladeeinrichtung (10), der Antriebseinrichtung (20), der Präparationseinrichtung (30) und/oder der Kühleinrichtung (50) verbunden ist.

11. Depositionsvorrichtung gemäß mindestens einem der vorhergehenden Ansprüche, die umfasst:
- eine Abbildungseinrichtung (70), insbesondere mit einer Kameraeinrichtung (71), einer Mikroskopieeinrichtung (72), einer Ultraschalleinrichtung (73), einer Stroboskopeinrichtung (74) und/oder einer Endoskopieeinrichtung (75),
- eine Navigationseinrichtung (80), die zur Positionierung der Depositionsvorrichtung (100) relativ zum Zielsubstrat (2) eingerichtet ist, und/oder
- eine Konditioniereinrichtung (90), insbesondere mit einer Beleuchtungseinrichtung (91) und/oder einer Temperiereinrichtung (92).

12. Verfahren zur Deposition biologischen Materials (1) in einem Zielsubstrat (2), welches biologische Zellen enthält, mit der Depositionsvorrichtung (100) gemäß mindestens einem der vorhergehenden Ansprüche, mit den Schritten:
- Beaufschlagung des biologischen Materials (1) mit der Antriebskraft, und
- Einbettung des biologischen Materials (1) im gefrorenen Zustand in das Zielsubstrat (2), wobei das biologische Material (1) durch die Wirkung der Antriebskraft in das Zielsubstrat (2) eingeführt wird, und
- das biologische Material (1) unter Wirkung der Antriebskraft eine Geschwindigkeit erhält, so dass seine kinetische Energie gleich der Verdrängungsarbeit ist, die es beim Eindringen in das Zielsubstrat (2) unter Verdrängung oder Zerstörung von Zellen im Zielsubstrat (2) bis zum Erreichen einer gewünschten Tiefe im Zielsubstrat (2) verrichtet.

13. Verfahren gemäß Anspruch 12, bei dem das biologische Material (1) biologische Zellen oder Zellgruppen enthält, die voneinander getrennt in das Zielsubstrat (2) eingebettet werden.

14. Verfahren gemäß mindestens einem der Ansprüche 12 bis 13, bei dem das biologische Material (1) mindestens eine biokompatible Zusatzsubstanz enthält.

15. Verfahren gemäß mindestens einem der Ansprüche 12 bis 14, bei dem das biologische Material (1) mindestens eine biologische Zelle und mindestens eine biokompatible Zusatzsubstanz enthält, die mindestens eine der folgenden Zusammensetzungen bilden:
- Zusammensetzung mit einer einzelnen Zelle, die von der Zusatzsubstanz umhüllt ist,
- Zusammensetzung mit einer Zellgruppe, die von der Zusatzsubstanz umhüllt ist,
- Zusammensetzung mit einer Zusatzsubstanz, die einen Hohlkörper bildet, in dem mindestens eine biologische Zelle angeordnet ist,
- Zusammensetzung mit einer Zusatzsubstanz, die von biologischen Zellen umhüllt ist, und
- Zusammensetzung mit der mindestens einen biologischen Zelle, der mindestens einen Zusatzsubstanz und einem festen Trägermaterial.

16. Verfahren gemäß mindestens einem der Ansprüche 12 bis 15, bei dem das Zielsubstrat (2) außerhalb eines Körpers eines biologischen Organismus angeordnet ist.

17. Verfahren gemäß Anspruch 16, bei dem das Zielsubstrat ein isoliertes biologisches Gewebe, ein Komposit aus biologischem Gewebe und nicht-biologischem Matrixmaterial, eine Zellkultur, eine Zell-Monoschicht, eine Zell-Multischicht, ein Matrixmaterial ohne biologische Zellen und/oder eine synthetische oder natürliche Zellkultureinrichtung umfasst.

18. Verfahren gemäß mindestens einem der Ansprüche 12 bis 17, bei dem aufeinanderfolgend verschiedene Zelltypen im Zielsubstrat (2) positioniert werden, und/oder bei dem das biologische Material (1) mit einem vorbestimmten geometrischen Muster im Zielsubstrat (2) positioniert wird.

19. Verfahren gemäß mindestens einem der Ansprüche 12 bis 18, bei dem das biologische Material (1) vor der Beaufschlagung mit der Antriebskraft in den gefrorenen Zustand überführt wird.

20. Verfahren gemäß Anspruch 19, mit dem Schritt:
- Einfrieren von Tropfen einer Zellsuspension, wobei hängende Tropfen der Zellsuspension eingefroren werden oder Tropfen der Zellsuspension auf einer hydrophoben Unterlage eingefroren werden.

21. Verfahren gemäß mindestens einem der Ansprüche 12 bis 20, bei dem das biologische Material (1) während der Beaufschlagung mit der Antriebskraft in den gefrorenen Zustand überführt wird.

22. Verfahren gemäß mindestens einem der Ansprüche 12 bis 21, mit mindestens einem der weiteren Schritte:
- Überwachung des Zielsubstrats (2) mit einem bildgebenden Verfahren, insbesondere unter Verwendung einer Kameraeinrichtung, von Ultraschall, NMR und/oder Mikroskopie,
- Erwärmung des Zielsubstrats (2) nach der Deposition des biologischen Materials (1), und
- Verringerung der Temperatur des Zielsubstrats (2) vor dem Einbetten des biologischen Materials (1).

## Claims

1. Deposition apparatus (100) which is designed to deposit biological material (1) in a frozen state in a target substrate (2), which includes biological cells, comprising a charging device (10) and a driving device (20), wherein
- the charging device (10) is designed to supply the biological material (1) to the driving device (20) and the driving device (20) is designed to apply a driving force to the biological material (1),
- the driving device (20) is formed for applying the driving force such that the biological material (1) receives kinetic energy by the effect of the driving force, so that it can penetrate into the target substrate (2) while displacing or destroying of cells present in the target substrate (2), wherein the kinetic energy is equal to a displacement work exerted by the biological material (1) until it reaches a desired depth in the target substrate (2), and
- the driving device (20) comprises a fluid drive with a gas, which is designed for creating the driving force by applying a gas pressure to the biological material,
**characterized by**
- a cooling device (50) which is designed to cool the biological material (1) and at least part of the driving device (20), wherein
- the cooling device (50) is designed to provide the gas for transmitting the driving force at a temperature below the freezing point of the biological material.

2. Deposition apparatus according to claim 1, which comprises:
- a preparation device (30) which is designed to supply the biological material to the charging device (10).

3. Deposition apparatus according to claim 2, in which the preparation device (30) contains a droplet generator, which is designed to generate droplets of the biological material, or a particle generator, which is designed to generate frozen droplets of the biological material.

4. Deposition apparatus according to at least one of the preceding claims, which comprises:
- a directing device (40) which is designed to orient a movement of the biological material towards the target substrate (2).

5. Deposition apparatus according to claim 4, in which the directing device (40) comprises a nozzle device (41) and/or a tool suitable for manual handling and having at least one hollow line (42).

6. Deposition apparatus according to at least one of the preceding claims, in which the charging device (10) comprises a sample injector (11) which is designed to introduce the biological material (1) into an acceleration line (22) of the driving device (20).

7. Deposition apparatus according to claim 6, in which the sample injector (11) comprises a droplet dispenser (13) which is designed to supply droplets of the biological material into the acceleration line (21) or a particle dispenser which is designed to supply particles of the frozen biological material into the acceleration line (21).

8. Deposition apparatus according to claim 7, in which the sample injector (11) comprises an injector cartridge (19) which is arranged replaceably in the acceleration line (21).

9. Deposition apparatus according to at least one of the preceding claims, in which the cooling device (50) is designed to cool at least part of the charging device (10) and/or at least part of the directing device (40).

10. Deposition apparatus according to at least one of the preceding claims, which comprises:
- a control circuit (60) which is connected to the control of the charging device (10), of the driving device (20), of the preparation device (30) and/or of the cooling device (50).

11. Deposition apparatus according to at least one of the preceding claims,
which comprises:
- an imaging device (70), in particular comprising a camera device (71), a microscopy device (72), an ultrasound device (73), a stroboscopy device (74) and/or an endoscopy device (75),
- a navigation device (80) which is designed to position the deposition apparatus (100) relative to the target substrate (2), and/or
- a conditioning device (90), in particular comprising an illumination device (91) and/or a temperature control device (92).

12. Method for depositing biological material (1) in a target substrate (2), which includes biological cells, comprising the steps:
- applying a driving force to the biological material (1), and
- embedding the biological material (1) in a frozen state in the target substrate (2), wherein the biological material (1) is introduced into the target substrate (2) under the effect of the driving force, and
- the biological material (1), under the effect of the driving force, has a velocity, so that it's kinetic energy is equal to the displacement work which it exerts during penetrating the target substrate (2) while displacing or destroying cells in the target substrate (2) by the biological material (1) until it reaches a desired depth in the target substrate (2).

13. Method according to claim 12, in which the biological material (1) contains biological cells or cell groups which are embedded separately from one another in the target substrate (2).

14. Method according to at least one of claims 12 to 13, in which the biological material (1) contains at least one biocompatible additional substance.

15. Method according to at least one of claims 12 to 14, in which the biological material (1) contains at least one biological cell and at least one biocompatible additional substance, which form at least one of the following compositions:
- composition containing one individual cell which is surrounded by the additional substance,
- composition containing a cell group which is surrounded by the additional substance,
- composition containing an additional substance which forms a hollow body, in which at least one biological cell is arranged,
- composition containing an additional substance which is surrounded by biological cells, and
- composition containing the at least one biological cell, the at least one additional substance and a solid carrier material.

16. Method according to at least one of claims 12 to 15, in which the target substrate (2) is arranged outside a body of a biological organism.

17. Method according to claim 16, in which the target substrate comprises an isolated biological tissue, a composite composed of biological tissue and non-biological matrix material, a cell culture, a cell monolayer, a cell multilayer, a matrix material without biological cells and/or a synthetic or natural cell culture device.

18. Method according to at least one of claims 12 to 17, in which different cell types are positioned in the target substrate (2) one after the other, and/or in which the biological material (1) is positioned in the target substrate (2) with a predetermined geometric pattern.

19. Method according to at least one of claims 12 to 18, in which the biological material (1) is transformed into the frozen state before the driving force is applied.

20. Method according to claim 19, comprising the step:
- freezing droplets of a cell suspension, wherein hanging droplets of the cell suspension are frozen or droplets of the cell suspension are frozen on a hydrophobic underlayer.

21. Method according to at least one of claims 12 to 20, in which the biological material (1) is transformed into the frozen state during or after the application of the driving force.

22. Method according to at least one of claims 12 to 21, comprising at least one of the further steps:
- monitoring the target substrate (2) by means of an imaging process, in particular by using a camera device, ultrasound, NMR and/or microscopy,
- heating the target substrate (2) after the biological material (1) has been deposited, and
- reducing the temperature of the target substrate (2) prior to the embedding of the biological material (1).

## Revendications

1. Dispositif de dépôt (100) qui est conçu pour le dépôt de matériau biologique (1) en un état congelé dans un substrat cible (2) qui contient des cellules biologiques, comprenant un système de chargement (10) et un système d'entraînement (20), dans lequel
- le système de chargement (10) est conçu pour la mise à disposition du matériau biologique (1) sur le système d'entraînement (20) et le système d'entraînement (20) est conçu pour l'exercice d'une force d'entraînement sur le matériau biologique (1),
- le système d'entraînement (20) est formé pour exercer la force d'entraînement de telle sorte que le matériau biologique (1) reçoive sous l'effet de la force d'entraînement, une énergie cinétique, de sorte qu'il puisse pénétrer dans le substrat cible (2) en déplaçant ou détruisant des cellules présentes dans le substrat cible, dans lequel l'énergie cinétique est égale à un travail de déplacement qui déplace le matériau biologique (1) jusqu'à ce qu'il atteigne une profondeur souhaitée dans le substrat cible (2), et
- le système d'entraînement (20) comprend un entraînement de fluide avec un gaz qui est conçu pour la génération de la force d'entraînement en exerçant une pression de gaz sur le matériau biologique,
**caractérisé par**
- un système de refroidissement (50) qui est conçu pour le refroidissement du matériau biologique (1) et au moins une partie du système d'entraînement (20), dans lequel
- le système de refroidissement (50) est conçu pour la mise à disposition du gaz pour la transmission de la force d'entraînement à une température inférieure au point de congélation du matériau biologique.

2. Dispositif de dépôt selon la revendication 1, qui comprend :
- un système de préparation (30) qui est conçu pour la mise à disposition du matériau biologique sur le système de chargement (10).

3. Dispositif de dépôt selon la revendication 2, où le système de préparation (30) contient un générateur de gouttes, qui est conçu pour la production des gouttes du matériau biologique, ou contient un générateur de particules, qui est conçu pour la production des gouttes gelées du matériau biologique.

4. Dispositif de dépôt selon au moins l'une des revendications précédentes, qui comprend :
- un système de pilotage (40) qui est conçu pour l'orientation d'un mouvement du matériau biologique vers le substrat cible (2).

5. Dispositif de dépôt selon la revendication 4, où le système de pilotage (40) présente un système de buses (41) et/ou un outil pouvant être manipulé manuellement, avec au moins un conduit creux (42).

6. Dispositif de dépôt selon au moins l'une des revendications précédentes, où le système de chargement (10) comprend un injecteur d'échantillon (11) qui est conçu pour l'introduction du matériau biologique (1) dans un conduit d'accélération (22) du système d'entraînement (20).

7. Dispositif de dépôt selon la revendication 6, où l'injecteur d'échantillon (11) présente soit un distributeur de gouttes (13) qui est conçu pour l'acheminement de gouttes du matériau biologique dans le conduit d'accélération (21), soit un distributeur de particules qui est conçu pour l'acheminement de particules du matériau biologique gelé dans le conduit d'accélération (22).

8. Système de dépôt selon la revendication 7, où l'injecteur d'échantillon (11) présente une cartouche d'injection (19) qui est disposée de façon échangeable dans le conduit d'accélération (21).

9. Système de dépôt selon au moins l'une des revendications précédentes, où le système de refroidissement (50) est conçu pour le refroidissement d'au moins une partie du système de chargement (10) et/ou au moins une partie du système de pilotage (40).

10. Système de dépôt selon au moins l'une des revendications précédentes, qui comprend :
- un circuit de réglage (60) qui est relié à la commande du système de chargement (10), du système d'entraînement (20), du système de préparation (30) et/ou du système de refroidissement (50).

11. Dispositif de dépôt selon au moins l'une des revendications précédentes, qui comprend :
- un système graphique (70), en particulier avec un système de caméra (71), un système de microscopie (72), un système échographique (73), un système de stroboscope (74) et/ou un système d'endoscopie (75),
- un système de navigation (80) qui est conçu pour le positionnement du dispositif de dépôt (100) par rapport au substrat cible (2), et/ou
- un système de conditionnement (90), en particulier avec un système d'éclairage (91) et/ou un système de régulation de température (92).

12. Procédé de dépôt de matériau biologique (1) dans un substrat cible (2) qui contient des cellules biologiques, avec le dispositif de dépôt (100) selon au moins l'une des revendications précédentes, avec les étapes suivantes :
- la sollicitation du matériau biologique (1) avec la force d'entraînement, et
- l'incorporation du matériau biologique (1) à l'état congelé dans le substrat cible (2), dans lequel le matériau biologique (1) est introduit sous l'effet de la force d'entraînement dans le substrat cible (2), et
- le matériau biologique (1) sous l'effet de la force d'entraînement obtient une vitesse de sorte que son énergie cinétique soit égale au travail de déplacement qui le déplace lors de la pénétration dans le substrat cible (2) par déplacement ou destruction de cellules dans le substrat cible (2) jusqu'à atteindre une profondeur souhaitée dans le substrat cible (2).

13. Procédé selon la revendication 12, où le matériau biologique (1) contient des cellules biologiques ou des groupes de cellules qui sont incorporées séparées les unes des autres dans le substrat cible (2).

14. Procédé selon au moins l'une des revendications 12 à 13, où le matériau biologique (1) contient au moins une substance formant un additif biocompatible.

15. Procédé selon au moins l'une des revendications 12 à 14, où le matériau biologique (1) contient au moins une cellule biologique et au moins une substance formant un additif biocompatible qui forment au moins l'une des compositions suivantes :
- la composition avec une cellule unique qui est enveloppée de la substance formant un additif,
- la composition avec un groupe de cellules qui est enveloppé de la substance formant un additif,
- la composition avec une substance formant un additif qui forme un corps creux dans lequel au moins une cellule biologique est disposée,
- la composition avec une substance formant un additif, qui est enveloppée de cellules biologiques, et
- la composition avec l'au moins une cellule biologique, l'au moins une substance formant un additif et un matériau formant un support solide.

16. Procédé selon au moins l'une des revendications 12 à 15, où le substrat cible (2) est disposé à l'extérieur d'un corps d'un organisme biologique.

17. Procédé selon la revendication 16, où le substrat cible comprend un tissu biologique isolé, un composite de tissu biologique et de matériau matriciel non biologique, une culture cellulaire, une monocouche de cellules, une multicouche de cellules, un matériau matriciel sans cellules biologiques et/ou un système de culture cellulaire synthétique ou naturel.

18. Procédé selon au moins l'une des revendications 12 à 17, où des types de cellules différents consécutifs sont positionnés dans le substrat cible (2), et/ou où le matériau biologique (1) est positionné avec un modèle géométrique prédéterminé dans le substrat cible (2).

19. Procédé selon au moins l'une des revendications 12 à 18, où le matériau biologique (1) est transformé, avant la soumission à l'action de la force d'entraînement, en l'état congelé.

20. Procédé selon la revendication 19, avec l'étape suivante :
- la congélation de gouttes d'une suspension cellulaire, dans lequel des gouttes suspendues de la suspension cellulaire sont congelées ou des gouttes de la suspension cellulaire sont congelées sur un support hydrophobe.

21. Procédé selon au moins l'une des revendications 12 à 20, où le matériau biologique (1) est transformé, pendant la soumission à l'action de la force d'entraînement, en l'état congelé.

22. Procédé selon au moins l'une des revendications 12 à 21, avec au moins l'une des autres étapes suivantes :
- la surveillance du substrat cible (2) avec un procédé d'imagerie, en particulier en utilisant un système de caméra, d'ultrasons, de RMN et/ou de microscopie,
- le chauffage du substrat cible (2) après le dépôt du matériau biologique (1), et
- la diminution de la température du substrat cible (2) avant l'incorporation du matériau biologique (1).
